# EUROPEAN PATENT APPLICATION

(11) **EP 3 683 206 A1**
(43) Date of publication of application: **22.07.2020**
(21) Application number: 18856441.3
(22) Date of filing: 11.09.2018
(51) Int. Cl.: C07D 209/12, C07D 401/12, A61K 31/404, A61P 35/00

(54) **DEUTERIUM ATOM-SUBSTITUTED INDOLE FORMAMIDE DERIVATIVE, PREPARATION METHOD THEREFOR, AND MEDICAL APPLICATIONS THEREOF**

(30) Priority: 12.09.2017 CN 201710817140
(71) Applicant: Jiangsu Hengrui Medicine Co., Ltd., Jiangsu 222047 (CN); Shanghai Hengrui Pharmaceutical Co., Ltd., Shanghai 200245 (CN)
(72) Inventor: LIU, Dong, Shanghai 200245 (CN); CHEN, Lei, Shanghai 200245 (CN); LU, Biao, Shanghai 200245 (CN); LIU, Suxing, Shanghai 200245 (CN); ZHANG, Rumin, Shanghai 200245 (CN); HE, Feng, Shanghai 200245 (CN); TAO, Weikang, Shanghai 200245 (CN)
(74) Representative: Potter Clarkson
(86) International application number: PCT/CN2018/105008
(87) International publication number: WO 2019/052440

(57) **Abstract**

A deuterium atom-substituted indole formamide derivative, a preparation method therefor, and medical applications thereof. Specifically, the present invention relates to a deuterium atom-substituted indole formamide derivative represented by general formula (I), a preparation method therefor, a pharmaceutical composition containing the derivative, and uses of the derivative serving as an ROR agonist and uses of the derivative in preventing and/or treating tumors or cancers, definitions of substituent groups in general formula (I) being same as definitions in the specification.

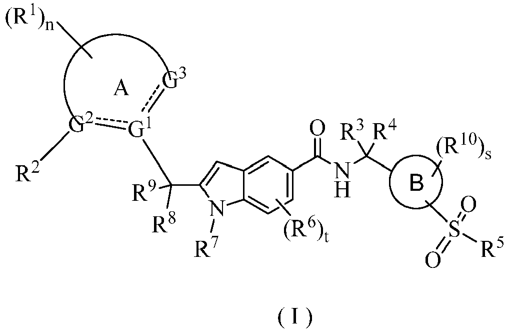

## Description

### FIELD OF THE INVENTION

The present invention belongs to the field of medicine, and relates to a deuterium (D) atom-substituted indole-formamide derivative, a method for preparing the same, and a use thereof in medicine. In particular, the present invention relates to a deuterium atom-substituted indole-formamide derivative of formula (I), a method for preparing the same, a pharmaceutical composition comprising the same, a use thereof as a ROR agonist, and a use thereof in the preparation of a medicament for preventing and/or treating tumor or cancer.

### BACKGROUND OF THE INVENTION

Retinoid-related orphan receptor (ROR) is a member of the nuclear receptor family, and is also a class of ligand-dependent transcription factors. It can regulate a variety of physiological and biochemical processes, including reproductive development, metabolism, immune system regulation and the like (Mech Dev. 1998 Jan, 70 (1-2: 147-53; EMBO J. 1998 Jul 15, 17(14): 3867-77). The ROR family includes three types: RORα, RORβ and RORγ (Curr Drug Targets Inflamm Allergy. 2004 Dec, 3(4): 395-412), among which, RORγ can be expressed in many tissues, including the thymus, liver, kidney, adipose, skeletal muscle and the like (Immunity. 1998 Dec, 9(6):797-806).

RORγ has two subtypes: RORγ1 and RORγt (RORγ2), among which, RORγ1 is expressed in many tissues, such as the thymus, muscle, kidney and liver, while RORγt is merely expressed in immune cells (Eur J Immunol. 1999 Dec, 29(12):4072-80). It has been reported in the literature that RORγt can regulate the survival of T cells during the differentiation of immune cells, and can activate and promote the differentiation of CD4+ and CD8+ cells into helper T cell 17 (Th17) and cytotoxic T cells (Tc17) (J Immunol. 2014 Mar 15, 192(6):2564-75). TH17 and Tc17 cells are a class of effector cells that promote inflammatory response, enhance acquired immune response and autoimmune response by secreting interleukin-17 (IL-17) and other inflammatory factors such as IL-21. In addition, existing studies have shown that the growth of transplanted tumor can be significantly inhibited by transplanting Th17 cells and Tc17 cells into tumor-bearing mice (J Immunol. 2010 Apr 15, 184(8):4215-27). Th17 can also recruit cytotoxic CD8+ T cells and natural killer cells to enter the tumor microenvironment, thereby killing tumor cells for an anti-tumor purpose (Blood. 2009 Aug 6, 114(6):1141-9; Clin Cancer Res. 2008 Jun 1, 14(11):3254-61). Therefore, activation of RORγt is likely to be a novel anti-tumor therapy.

At present, pharmaceutical companies have developed agonists of RORγt, such as the small molecule drug LYC-54143 developed by Lycera Corp. Pre-clinical studies have shown that LYC-54143 inhibits tumor growth through two distinct pathways, and exhibits a superior anticancer activity. Firstly, LYC-54143 activates RORγt to regulate the differentiation of Th17 and Tc17 cells through traditional pathways, promote the expression of other cytokines such as IL-17, and increase T cell activity. Moreover, activated RORγt can regulate the expression of various genes in the immune system, inhibit the expression of PD-1 in cellular checkpoint receptors, thereby reducing immunosuppression and increasing anticancer activity (Oncoimmunology. 2016 Nov 4, 5(12): e1254854; ACS Chem Biol. 2016 Apr 15, 11(4):1012-8). Although this small molecule agonist has currently entered clinical phase II, there are still very few drugs related to this target agonist, and there are no drugs on the market. Disclosed patent applications include, for example, WO2015171558, WO2008152260, WO2007068580, WO2007068579, WO2005056516, WO2005056510, WO2005066116 and WO00228810. There is still a need to continue to develop novel and more efficient RORγt agonists in order to provide patients with novel and effective anticancer drugs.

The inventors have designed a deuterium atom-substituted indole-formamide compound having a structure represented by formula (I), wherein the resence of a deuterium atom in the substituent allows the compound of the present invention to achieve unexpected pharmacokinetic absorption activity and pharmacological efficacy. Meanwhile, when there is a large substituent (for example trifluoromethyl) in the ortho position of ring A, the compound will show a significant agonistic effect on ROR. The present invention also provides a pharmacodynamic test, in which the compound of the present invention exhibits a good antitumor activity when being administered alone. In addition, the compound of the present invention exhibits a synergistic effect when being administered in combination with a PD-1 antibody, leading to a novel way of improving the efficacy of immunotherapy.

### SUMMARY OF THE INVENTION

The object of the present invention is to provide a compound of formula (I): 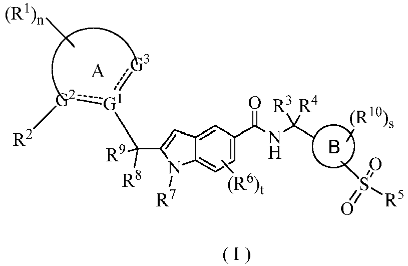 or a tautomer, mesomer, racemate, enantiomer, diastereomer thereof, or mixture thereof, or a pharmaceutically acceptable salt thereof,
wherein:
----- is a double bond or single bond;
G¹, G² and G³ are identical or different and are each independently selected from the group consisting of C, CH, CH₂ and N;
ring A is selected from the group consisting of aryl, heteroaryl, cycloalkyl and heterocyclyl;
ring B is an aryl or heteroaryl;
each R¹ is identical or different and each is independently selected from the group consisting of H atom, D atom, halogen, alkyl, haloalkyl, alkoxy, haloalkoxy, cyano, amino, nitro, hydroxy and hydroxyalkyl;
R² is a haloalkyl;
R³ and R⁴ are identical or different and are each independently selected from the group consisting of H atom, D atom, halogen, alkyl, haloalkyl, alkoxy, haloalkoxy, hydroxyalkyl, cyano, amino, nitro, hydroxy, cycloalkyl, heterocyclyl, aryl and heteroaryl, wherein the alkyl, hydroxyalkyl, cycloalkyl, heterocyclyl, aryl and heteroaryl are each independently optionally substituted by one or more substituents selected from the group consisting of D atom, hydroxy, halogen, alkyl, amino and -OR¹¹;
R⁵ is selected from the group consisting of H atom, alkyl, haloalkyl, amino, hydroxy, hydroxyalkyl, cycloalkyl, heterocyclyl, aryl and heteroaryl, wherein the alkyl, cycloalkyl, heterocyclyl, aryl and heteroaryl are optionally substituted by one or more substituents selected from the group consisting of D atom, halogen, hydroxy, cycloalkyl and heterocyclyl;
each R⁶ is identical or different and each is independently selected from the group consisting of H atom, D atom, halogen, alkyl, haloalkyl, alkoxy, haloalkoxy, cyano, amino, nitro, hydroxy, hydroxyalkyl, cycloalkyl, heterocyclyl, aryl and heteroaryl;
R⁷ is selected from the group consisting of H atom, alkyl, haloalkyl, cycloalkyl, heterocyclyl, aryl and heteroaryl, wherein the alkyl, cycloalkyl, heterocyclyl, aryl and heteroaryl are optionally substituted by one or more substituents selected from the group consisting of D atom, halogen, hydroxy, amino, cyano, nitro, alkoxy, haloalkoxy, -OR¹¹, -C(O)R¹¹, -C(O)OR¹¹, -NR¹²R¹³, -C(O)NR¹²R¹³, -S(O)ₘR¹¹, cycloalkyl and heterocyclyl;
R⁸ and R⁹ are identical or different and are each independently selected from the group consisting of H atom, D atom, halogen, alkyl, haloalkyl, alkoxy, cyano, amino, nitro, hydroxy and hydroxyalkyl;
each R¹⁰ is identical or different and each is independently selected from the group consisting of H atom, D atom, halogen, alkyl, haloalkyl, alkoxy, haloalkoxy, cyano, amino, nitro, hydroxy, hydroxyalkyl, cycloalkyl, heterocyclyl, aryl and heteroaryl;
R¹¹ is selected from the group consisting of H atom, D atom, alkyl, haloalkyl, hydroxyalkyl, cycloalkyl and heterocyclyl, wherein the alkyl, hydroxyalkyl, cycloalkyl and heterocyclyl are optionally substituted by one or more substituents selected from the group consisting of D atom, halogen, hydroxy, cycloalkyl and heterocyclyl;
R¹² and R¹³ are identical or different and are each independently selected from the group consisting of H atom, D atom, alkyl, haloalkyl, hydroxy and hydroxyalkyl, wherein the alkyl and hydroxyalkyl are optionally substituted by one or more substituents selected from the group consisting of D atom, halogen, hydroxy, cycloalkyl and heterocyclyl;
provided that the compound of formula (I) comprises at least one D atom, in particular, at least one of substituents R³, R⁴, R⁵, R⁷, R⁸ and R⁹ comprises at least one D atom;
m is 0, 1 or 2;
n is 0, 1, 2, 3 or 4;
s is 0, 1, 2 or 3; and
t is 0, 1, 2 or 3.

In a preferred embodiment of the present invention, in the compound of formula (I), ring A is selected from the group consisting of phenyl, pyridyl, imidazolyl, pyrazolyl, piperidinyl and morpholinyl; and ring B is a phenyl or pyridyl.

In a preferred embodiment of the present invention, in the compound of formula (I), 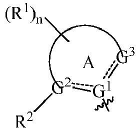 is selected from the group consisting of 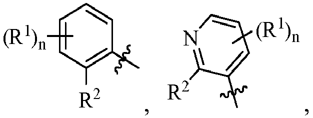 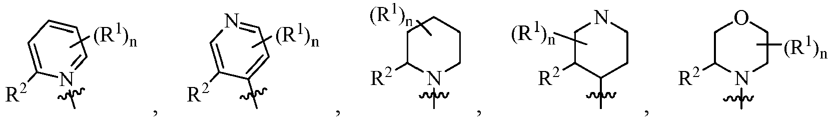 and

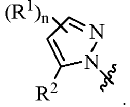

In a preferred embodiment of the present invention, the compound of formula (I) is a compound of formula (II): 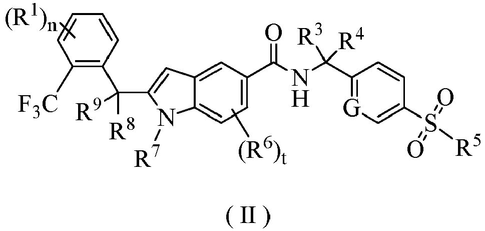 wherein:
G is CH or N; and
R¹, R³∼R⁹, n and t are as defined in formula (I).

In a preferred embodiment of the present invention, in the compound of formula (I), R⁴ is a H atom or D atom; R³ is selected from the group consisting of H atom, D atom and alkyl, wherein the alkyl is optionally substituted by one or more substituents selected from the group consisting of D atom, hydroxy, halogen, amino and -OR¹¹; and R¹¹ is as defined in formula (I).

In a preferred embodiment of the present invention, in the compound of formula (I), R⁷ is selected from the group consisting of alkyl, haloalkyl, cycloalkyl and heterocyclyl, wherein the alkyl is optionally substituted by one or more substituents selected from the group consisting of D atom, halogen, hydroxy, amino, cyano, nitro, alkoxy, haloalkoxy and hydroxyalkyl.

In a preferred embodiment of the present invention, in the compound of formula (I), R⁸ and R⁹ are identical or different and are each independently selected from the group consisting of H atom and D atom.

In a preferred embodiment of the present invention, the compound of formula (I) is a compound of formula (III): 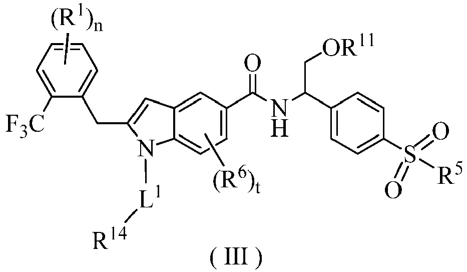 wherein:
L¹ is an alkylene, wherein the alkylene is optionally substituted by one or more substituents selected from the group consisting of halogen and D atom;
R¹⁴ is selected from the group consisting of D atom, halogen, hydroxy, amino, cyano, nitro, alkoxy, haloalkoxy and hydroxyalkyl; and
R¹, R⁵, R⁶, R¹¹, n and t are as defined in formula (I).

In a preferred embodiment of the present invention, the compound of formula (I) is a compound of formula (IV): 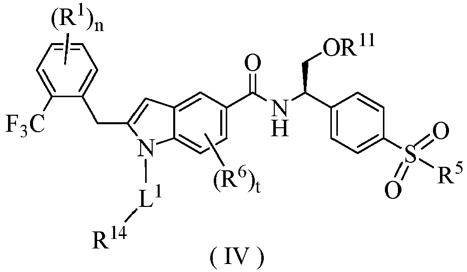 wherein:
L¹ is an alkylene, wherein the alkylene is optionally substituted by one or more substituents selected from the group consisting of halogen and D atom;
R¹⁴ is selected from the group consisting of D atom, halogen, hydroxy, amino, cyano, nitro, alkoxy, haloalkoxy and hydroxyalkyl; and
R¹, R⁵, R⁶, R¹¹, n and t are as defined in formula (I).

In a preferred embodiment of the present invention, in the compound of formula (I), each R¹ is identical or different and each is independently selected from the group consisting of H atom, D atom, halogen and alkyl.

In a preferred embodiment of the present invention, in the compound of formula (I), R⁵ is an alkyl, wherein the alkyl is optionally substituted by one or more substituents selected from the group consisting of D atom, halogen and hydroxy.

In a preferred embodiment of the present invention, in the compound of formula (I), each R⁶ is identical or different and each is independently selected from the group consisting of H atom, D atom and halogen.

Typical compounds of formula (I) include, but are not limited to:

| Example No. | Structure and name of the compound |
|---|---|
| 1 | 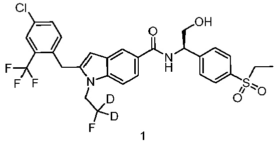 |
| | 2-[[4-Chloro-2-(trifluoromethyl)phenyl]methyl]-1-(2,2-dideutero-2-fluoro -ethyl)-*N*-[(1*R*)-1-(4-ethylsulfonylphenyl)-2-hydroxy-ethyl]indole-5-carb oxamide **1** |
| 2 | 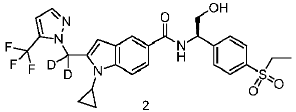 |
| | 1-Cyclopropyl-2-[dideutero-[5-(trifluoromethyl)pyrazol-1-yl]methyl]-*N*-[( 1*R*)-1-(4-ethylsulfonylphenyl)-2-hydroxy-ethyl]indole-5-carboxamide **2** |
| 3 | 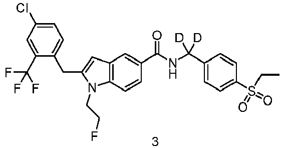 |
| | 2-[[4-Chloro-2-(trifluoromethyl)phenyl]methyl]-*N*-[dideutero-(4-ethylsulf onylphenyl)methyl]-1-(2-fluoroethyl)indole-5-carboxamide **3** |
| 4 | 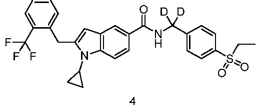 |
| | 1-Cyclopropyl-*N*-[dideutero-(4-ethylsulfonylphenyl)methyl]-2-[[2-(trifluo romethyl)phenyl]methyl]indole-5-carboxamide **4** |
| 5 | 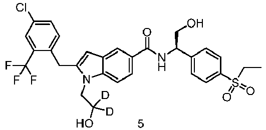 |
| | 2-[[4-Chloro-2-(trifluoromethyl)phenyl]methyl]-1-(2,2-dideutero-2-hydro xy-ethyl)-*N*-[(1*R*)-1-(4-ethylsulfonylphenyl)-2-hydroxy-ethyl]indole-5-ca rboxamide **5** |
| 6 | 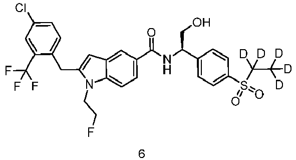 |
| | 2-[[4-Chloro-2-(trifluoromethyl)phenyl]methyl]-1-(2-fluoroethyl)-*N*-[(1*R*) -2-hydroxy-1-[4-(1,1,2,2,2-pentadeuteroethylsulfonyl)phenyl]ethyl]indole -5-carboxamide **6** |
| 7 | 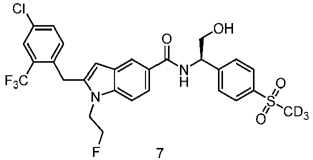 |
| | 2-[[4-Chloro-2-(trifluoromethyl)phenyl]methyl]-1-(2-fluoroethyl)-*N*-[(1*R*) -2-hydroxy-1-[4-(trideuteromethylsulfonyl)phenyl]ethyl]indole-5-carboxa mide **7** |
| 8 | 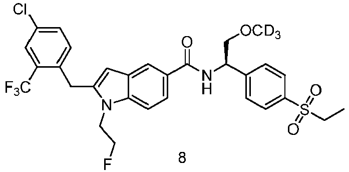 |
| | 2-[[4-Chloro-2-(trifluoromethyl)phenyl]methyl]-*N*-[(1*R*)-1-(4-ethylsulfon ylphenyl)-2-(trideuteromethoxy)ethyl]-1-(2-fluoroethyl)indole-5-carboxa mide **8** |
| 9 | 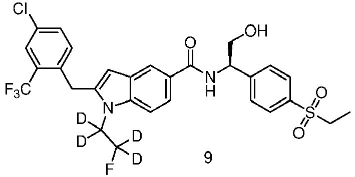 |
| | 2-[[4-Chloro-2-(trifluoromethyl)phenyl]methyl]-*N*-[(1*R*)-1-(4-ethylsulfon ylphenyl)-2-hydroxyethyl]-1-(1,1,2,2-tetradeutero-2-fluoro-ethyl)indole-5 -carboxamide **9** |
| 10 | 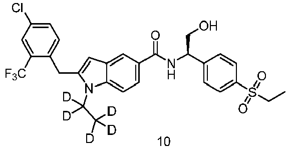 |
| | 2-[[4-Chloro-2-(trifluoromethyl)phenyl]methyl]-*N*-[(1*R*)-1-(4-ethylsulfon ylphenyl)-2-hydroxyethyl]-1-(1,1,2,2,2-pentadeuteroethyl)indole-5-carboxamide **10** |
| 11 | 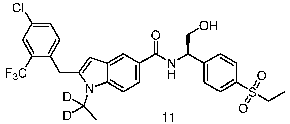 |
| | 2-[[4-Chloro-2-(trifluoromethyl)phenyl]methyl]-1-(1,1-dideuteroethyl)-*N-*[(1*R*)-1-(4-ethylsulfonylphenyl)-2-hydroxyethyl]indole-5-carboxamide **11** |
| 12 | 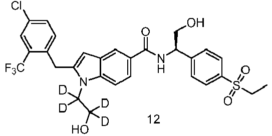 |
| | 2-[[4-Chloro-2-(trifluoromethyl)phenyl]methyl]-*N*-[(1*R*)-1-(4-ethylsulfon ylphenyl)-2-hydroxyethyl]-1-(1,1,2,2-tetradeutero-2-hydroxy-ethyl)indole -5-carboxamide **12** |
| 13 | 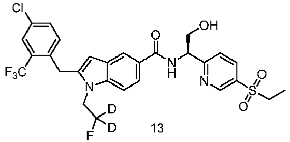 |
| | 2-[[4-Chloro-2-(trifluoromethyl)phenyl]methyl]-1-(2,2-dideutero-2-fluoro -ethyl)-*N*-[(1*R*)-1-(5-ethylsulfonyl-2-pyridinyl)-2-hydroxy-ethyl]indole-5-carboxamide **13** |
| 14 | 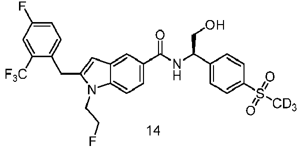 |
| | 1-(2-Fluoroethyl)-2-[[4-fluoro-2-(trifluoromethyl)phenyl]methyl]-*N*-[(1*R*) -2-hydroxy-1-[4-(trideuteromethylsulfonyl)phenyl]ethyl]indole-5-carboxa mide **14** |
| 15 | 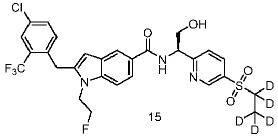 |
| | 2-[[4-Chloro-2-(trifluoromethyl)phenyl]methyl]-1-(2-fluoroethyl)-*N*-[(1*R*) -2-hydroxy-1-[5-(1,1,2,2,2-pentadeuteroethylsulfonyl)-2-pyridinyl]ethyl]i ndole-5-carboxamide **15** |
| 16 | 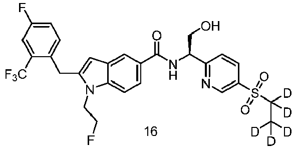 |
| | 1-(2-Fluoroethyl)-2-[[4-fluoro-2-(trifluoromethyl)phenyl]methyl]-*N*-[(1*R*)-2-hydroxy-1-[5-(1,1,2,2,2-pentadeuteroethylsulfonyl)-2-pyridinyl]ethyl]i ndole-5-carboxamide **16** |
| 17 | 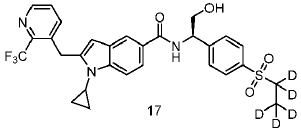 |
| | 1-Cyclopropyl-*N*-[(1*R*)-2-hydroxy-1-[4-(1,1,2,2,2-pentadeuteroethylsulfo nyl)phenyl]ethyl]-2-[[2-(trifluoromethyl)-3-pyridinyl]methyl]indole-5-car boxamide **17** |
| 18 | 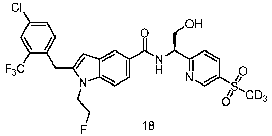 |
| | 2-[[4-Chloro-2-(trifluoromethyl)phenyl]methyl]-1-(2-fluoroethyl)-*N*-[(1*R*) -2-hydroxy-1-[5-(trideuteromethylsulfonyl)-2-pyridinyl]ethyl]indole-5-ca rboxamide **18** |
| 19 | 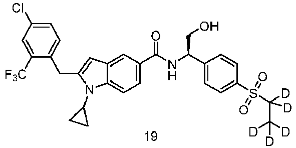 |
| | 2-[4-Chloro-2-(trifluoromethyl)phenyl]methyl]-1-cyclopropyl-*N*-[(1*R*)-2-hydroxy-1-[4-(1,1,2,2,2-pentadeuteroethylsulfonyl)phenyl]ethyl]indole-5-carboxamide **19** |
| 20 | 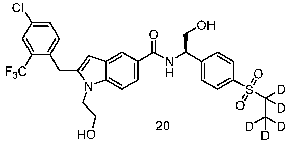 |
| | 2-[[4-Chloro-2-(trifluoromethyl)phenyl]methyl]-1-(2-hydroxyethyl)-*N*-[(1 *R*)-2-hydroxy-1-[4-(1,1,2,2,2-pentadeuteroethylsulfonyl)phenyl]ethyl]ind ole-5-carboxamide **20** |
| 21 | 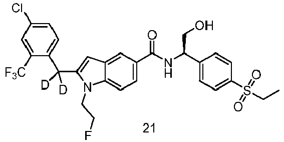 |
| | 2-[[4-Chloro-2-(trifluoromethyl)phenyl]-dideutero-methyl]-*N*-[(1*R*)-1-(4-ethylsulfonylphenyl)-2-hydroxy-ethyl]-1-(2-fluoroethyl)indole-5-carboxa mide **21** |
| 22 | 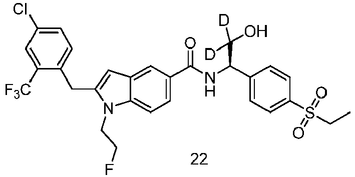 |
| | 2-[[4-Chloro-2-(trifluoromethyl)phenyl]methyl]-*N*-[(1*R*)-2,2-dideutero-1-(4-ethylsulfonylphenyl)-2-hydroxyethyl]-1-(2-fluoroethyl)indole-5-carbo xamide **22** |
| 23 | 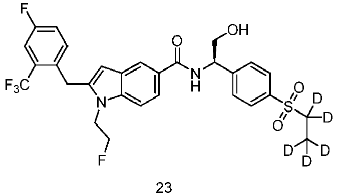 |
| | 1-(2-Fluoroethyl)-2-[[4-fluoro-2-(trifluoromethyl)phenyl]methyl]-*N*-[(1*R*) -2-hydroxy-1-[4-(1,1,2,2,2-pentadeuteroethylsulfonyl)phenyl]ethyl]indole -5-carboxamide **23** |
| 24 | 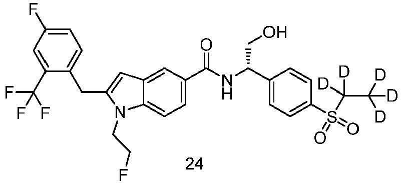 |
| | 1-(2-Fluoroethyl)-2-[[4-fluoro-2-(trifluoromethyl)phenyl]methyl]-*N*-[(1*S*) -2-hydroxy-1-[4-(1,1,2,2,2-pentadeuteroethylsulfonyl)phenyl]ethyl]indole -5-carboxamide **24** |

or a tautomer, mesomer, racemate, enantiomer, diastereomer thereof, or mixture thereof, or a pharmaceutically acceptable salt thereof.

In another aspect, the present invention relates to a compound of formula (V), which is an intermediate for preparing the compound of formula (I), 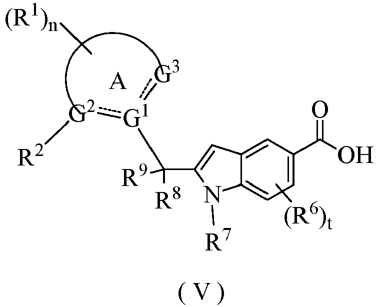 or a tautomer, mesomer, racemate, enantiomer, diastereomer thereof, or mixture thereof, or a pharmaceutically acceptable salt thereof,
wherein:
----- is a double bond or single bond;
G¹, G² and G³ are identical or different and are each independently selected from the group consisting of C, CH, CH₂ and N;
ring A is selected from the group consisting of aryl, heteroaryl, cycloalkyl and heterocyclyl;
each R¹ is identical or different and each is independently selected from the group consisting of H atom, D atom, halogen, alkyl, haloalkyl, alkoxy, haloalkoxy, cyano, amino, nitro, hydroxy and hydroxyalkyl;
R² is a haloalkyl;
each R⁶ is identical or different and each is independently selected from the group consisting of H atom, D atom, halogen, alkyl, haloalkyl, alkoxy, haloalkoxy, cyano, amino, nitro, hydroxy, hydroxyalkyl, cycloalkyl, heterocyclyl, aryl and heteroaryl;
R⁷ is selected from the group consisting of H atom, alkyl, haloalkyl, cycloalkyl, heterocyclyl, aryl and heteroaryl, wherein the alkyl, cycloalkyl, heterocyclyl, aryl and heteroaryl are optionally substituted by one or more substituents selected from the group consisting of D atom, halogen, hydroxy, amino, cyano, nitro, alkoxy, haloalkoxy, -OR¹¹, -C(O)R¹¹, -C(O)OR¹¹, -NR¹²R¹³, -C(O)NR¹²R¹³, -S(O)ₘR¹¹, cycloalkyl and heterocyclyl;
R⁸ and R⁹ are identical or different and are each independently selected from the group consisting of H atom, D atom, halogen, alkyl, haloalkyl, alkoxy, cyano, amino, nitro, hydroxy and hydroxyalkyl;
R¹¹ is selected from the group consisting of H atom, D atom, alkyl, haloalkyl, hydroxyalkyl, cycloalkyl and heterocyclyl, wherein the alkyl, hydroxyalkyl, cycloalkyl and heterocyclyl are optionally substituted by one or more substituents selected from the group consisting of D atom, halogen, hydroxy, cycloalkyl and heterocyclyl;
R¹² and R¹³ are identical or different and are each independently selected from the group consisting of H atom, D atom, alkyl, haloalkyl, hydroxy and hydroxyalkyl, wherein the alkyl and hydroxyalkyl are optionally substituted by one or more substituents selected from the group consisting of D atom, halogen, hydroxy, cycloalkyl and heterocyclyl;
m is 0, 1 or 2;
n is 0, 1, 2, 3 or 4; and
t is 0, 1, 2 or 3.

In a preferred embodiment of the present invention, in the compound of formula (V), at least one of substituents R⁷, R⁸ and R⁹ comprises one or more D atoms.

Typical compounds of formula (V) include, but are not limited to:

| Example No. | Structure and name of the compound |
|---|---|
| 1k | 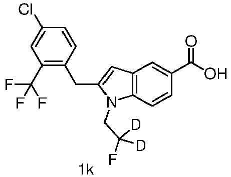 |
| | 2-[[4-Chloro-2-(trifluoromethyl)phenyl]methyl]-1-(2,2-dideutero-2-fluoro -ethyl)indole-5-carboxylic acid **1k** |
| 2f | 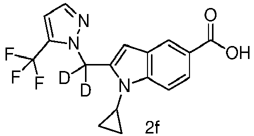 |
| | 1-Cyclopropyl-2-[dideutero-[5-(trifluoromethyl)pyrazol-1-yl]methyl]indo le-5-carboxylic acid **2f** |
| 3g | 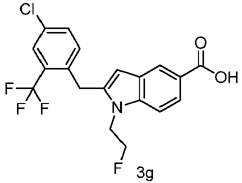 |
| | 2-(4-Chloro-2-(trifluoromethyl)benzyl)-1-(2-fluoroethyl)-1*H*-indole-5-car boxylic acid **3g** |
| 4d | 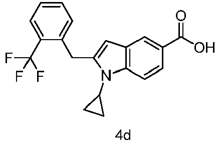 |
| | 1-Cyclopropyl-2-(2-(trifluoromethyl)benzyl)-1*H*-indole-5-carboxylic acid **4d** |
| 5a | 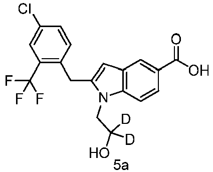 |
| | 2-[[4-Chloro-2-(trifluoromethyl)phenyl]methyl]-1-(2,2-dideutero-2-hydro xy-ethyl)indole-5-carboxylic acid **5a** |
| 23d | 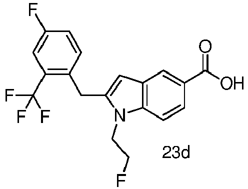 |
| | 2-(4-Fluoro-2-(trifluoromethyl)benzyl)-1-(2-fluoroethyl)-1*H*-indole-5-car boxylic acid **23d** |

or a tautomer, mesomer, racemate, enantiomer, diastereomer thereof, or mixture thereof, or a pharmaceutically acceptable salt thereof.

In another aspect, the present invention relates to a method for preparing the compound of formula (I), comprising a step of: 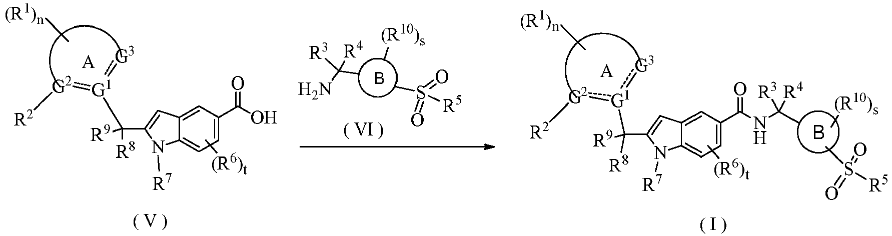 subjecting a compound of formula (V) and a compound of formula (VI) or a pharmaceutically acceptable salt thereof to a condensation reaction to obtain the compound of formula (I),
wherein:
-----, ring A, ring B, G¹∼G³, R¹∼R¹⁰, n, s and t are as defined in formula (I).

In another aspect, the present invention relates to a method for preparing the compound of formula (II), comprising a step of: 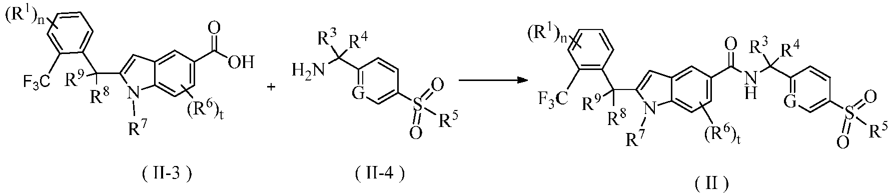 subjecting a compound of formula (II-3) and a compound of formula (II-4) or a pharmaceutically acceptable salt thereof to a condensation reaction to obtain the compound of formula (II),
wherein:
Q R¹, R³∼R⁹, n and t are as defined in formula (II).

In another aspect, the present invention relates to a method for preparing the compound of formula (III), comprising a step of: 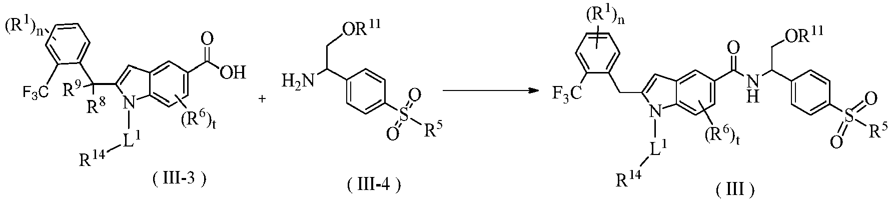 subjecting a compound of formula (III-3) and a compound of formula (III-4) or a pharmaceutically acceptable salt thereof to a condensation reaction to obtain the compound of formula (III),
wherein:
R¹, R⁵, R⁶, R¹¹, R¹⁴, L¹, n and t are as defined in formula (III); and R⁸ and R⁹ are as defined in formula (I).

In another aspect, the present invention relates to a method for preparing the compound of formula (IV), comprising a step of: 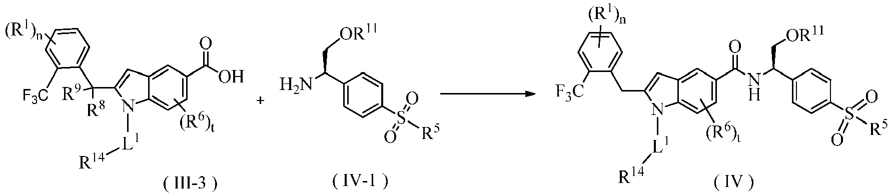 subjecting a compound of formula (III-3) and a compound of formula (IV-1) or a pharmaceutically acceptable salt thereof to a condensation reaction to obtain the compound of formula (IV),
wherein:
R¹, R⁵, R⁶, R¹¹, R¹⁴, L¹, n and t are as defined in formula (IV); and R⁸ and R⁹ are as defined in formula (I).

In another aspect, the present invention relates to a pharmaceutical composition comprising a therapeutically effective amount of the compound of formula (I), or a tautomer, mesomer, racemate, enantiomer, diastereomer thereof, or mixture thereof, or a pharmaceutically acceptable salt thereof, and one or more pharmaceutically acceptable carriers, diluents or excipients. The present invention also relates to a method for preparing the pharmaceutical composition, comprising a step of mixing the compound of formula (I), or a tautomer, mesomer, racemate, enantiomer, diastereomer thereof, or mixture thereof, or a pharmaceutically acceptable salt thereof with the pharmaceutically acceptable carrier(s), diluent(s) or excipient(s). In an embodiment of the present invention, the pharmaceutical composition further comprises an anti-PD-1 antibody, preferably an anti-mouse PD-1 antibody.

The present invention further relates to a use of the compound of formula (I), or a tautomer, mesomer, racemate, enantiomer, diastereomer thereof, or mixture thereof, or a pharmaceutically acceptable salt thereof, or the pharmaceutical composition comprising the same in the preparation of a ROR agonist.

The present invention further relates to a use of the compound of formula (I), or a tautomer, mesomer, racemate, enantiomer, diastereomer thereof, or mixture thereof, or a pharmaceutically acceptable salt thereof, or the pharmaceutical composition comprising the same, in particular as a ROR agonist, in the preparation of a medicament for preventing and/or treating tumor or cancer.

The present invention further relates to a use of the compound of formula (I), or a tautomer, mesomer, racemate, enantiomer, diastereomer thereof, or mixture thereof, or a pharmaceutically acceptable salt thereof (as a ROR agonist), or the pharmaceutical composition comprising the same, in combination with an anti-PD-1 antibody in the preparation of a medicament for preventing and/or treating tumor or cancer.

The present invention further relates to the compound of formula (I), or a tautomer, mesomer, racemate, enantiomer, diastereomer thereof, or mixture thereof, or a pharmaceutically acceptable salt thereof, or the pharmaceutical composition comprising the same, for use as a medicament.

The present invention also relates to the compound of formula (I), or a tautomer, mesomer, racemate, enantiomer, diastereomer thereof, or mixture thereof, or a pharmaceutically acceptable salt thereof, or the pharmaceutical composition comprising the same, for use as a ROR agonist.

The present invention also relates to the compound of formula (I), or a tautomer, mesomer, racemate, enantiomer, diastereomer thereof, or mixture thereof, or a pharmaceutically acceptable salt thereof, or the pharmaceutical composition comprising the same, for use in particular as a ROR agonist in preventing and/or treating tumor or cancer.

The present invention also relates to the combination of the compound of formula (I), or a tautomer, mesomer, racemate, enantiomer, diastereomer thereof, or mixture thereof, or a pharmaceutically acceptable salt thereof, or the pharmaceutical composition comprising the same and an anti-PD-1 antibody, for use in preventing and/or treating tumor or cancer.

The present invention also relates to a method for preventing and/or treating tumor or cancer, comprising a step of administrating to a patient in need thereof a therapeutically effective dose of the compound of formula (I), or a tautomer, mesomer, racemate, enantiomer, diastereomer thereof, or mixture thereof, or a pharmaceutically acceptable salt thereof, or the pharmaceutical composition comprising the same as a ROR agonist.

The present invention also relates to a method for preventing and/or treating tumor or cancer, comprising a step of administrating to a patient in need thereof a therapeutically effective dose of the compound of formula (I), or a tautomer, mesomer, racemate, enantiomer, diastereomer thereof, or mixture thereof, or a pharmaceutically acceptable salt thereof, or the pharmaceutical composition comprising the same and an anti-PD-1 antibody.

The tumor or cancer of the present invention is selected from the group consisting of non-Hodgkin's lymphoma, diffuse large B-cell lymphoma, follicular lymphoma, synovial sarcoma, breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, papillary renal carcinoma, head and neck tumor, leukemia, lymphoma, myeloma and non-small cell lung cancer.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such a composition can contain one or more ingredient(s) selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the manufacture of tablets. These excipients can be inert excipients, granulating agents, disintegrating agents, binders and lubricants. The tablet can be uncoated or coated by means of a known technique to mask drug taste or delay the disintegration and absorption of the active ingredient in the gastrointestinal tract, thereby providing sustained release over a long period of time.

An oral formulation can also be provided as soft gelatin capsules in which the active ingredient is mixed with an inert solid diluent, or the active ingredient is mixed with a water-soluble carrier or an oil medium.

An aqueous suspension contains the active ingredient in admixture with excipients suitable for the manufacture of an aqueous suspension. Such excipients are suspending agents, dispersants or wetting agents. The aqueous suspension can also contain one or more preservatives, one or more colorants, one or more flavoring agents, and one or more sweeteners.

An oil suspension can be formulated by suspending the active ingredient in a vegetable oil or mineral oil. The oil suspension can contain a thickener. The aforementioned sweeteners and flavoring agents can be added to provide a palatable formulation. These compositions can be preserved by adding an antioxidant.

The pharmaceutical composition of the present invention can also be in the form of an oil-in-water emulsion. The oil phase can be a vegetable oil, or a mineral oil, or a mixture thereof. Suitable emulsifying agents can be naturally occurring phospholipids. The emulsion can also contain a sweetening agent, flavoring agent, preservative and antioxidant. Such a formulation can also contain a demulcent, preservative, colorant and antioxidant.

The pharmaceutical composition of the present invention can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. The injectable solution or micro-emulsion can be introduced into a patient's bloodstream by local bolus injection. Alternatively, the solution and micro-emulsion are preferably administered in a manner that maintains a constant circulating concentration of the compound of the present invention. In order to maintain this constant concentration, a continuous intravenous delivery device can be used. An example of such a device is Deltec CADD-PLUS. TM. 5400 intravenous injection pump.

The pharmaceutical composition of the present invention can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium. For this purpose, any blended fixed oil can be used. In addition, fatty acids can also be used to prepare injections.

The compound of the present invention can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

### DEDINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is one or more groups independently selected from the group consisting of deuterium atom, alkyl, alkenyl, alkynyl, alkoxy, alkylthio, alkylamino, halogen, thiol, hydroxy, nitro, cyano, cycloalkyl, heterocycloalkyl, aryl, heteroaryl, cycloalkoxy, heterocycloalkoxy, cycloalkylthio, heterocyclylthio, oxo, carboxyl, carboxylate, -OR¹¹, -C(O)R¹¹, -C(O)OR¹¹, -NR¹²R¹³, -C(O)NR¹²R¹³ and -S(O)ₘR¹¹.

The term "alkylene" refers to a saturated linear or branched aliphatic hydrocarbon group having two residues derived from the removal of two hydrogen atoms from the same carbon atom or two different carbon atoms of the parent alkane. The linear or branched alkylene has 1 to 20 carbon atoms, preferably 1 to 12 carbon atoms, and more preferably 1 to 6 carbon atoms. Non-limiting examples of alkylene groups include, but are not limited to, methylene (-CH₂-), 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), 1,5-pentylene (-CH₂CH₂CH₂CH₂CH₂-), and the like. The alkylene group can be substituted or unsubstituted. When substituted, the substituent group(s) can be substituted at any available connection point. The substituent group(s) is one or more groups independently optionally selected from the group consisting of deuterium atom, alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heteroalkoxy, cycloalkylthio, heterocyclylthio, oxo, -OR¹¹, -C(O)R¹¹, -C(O)OR¹¹, -NR¹²R¹³, -C(O)NR¹²R¹³ and -S(O)ₘR¹¹.

The term "alkoxy" refers to an -O-(alkyl) or an -O-(unsubstituted cycloalkyl) group, wherein the alkyl and cycloalkyl are as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is one or more group(s) independently selected from the group consisting of deuterium atom, alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl, carboxylate, -OR¹¹, -C(O)R¹¹, -C(O)OR¹¹, -NR¹²R¹³, -C(O)NR¹²R¹³ and -S(O)ₘR¹¹.

The term "cycloalkyl" refers to a saturated or partially unsaturated monocyclic or polycyclic hydrocarbon substituent group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

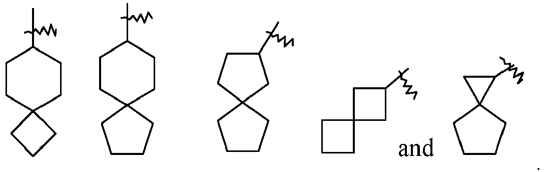

The term "fused cycloalkyl" refers to a 5 to 20 membered all-carbon polycyclic group, wherein each ring in the system shares an adjacent pair of carbon atoms with another ring, wherein one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

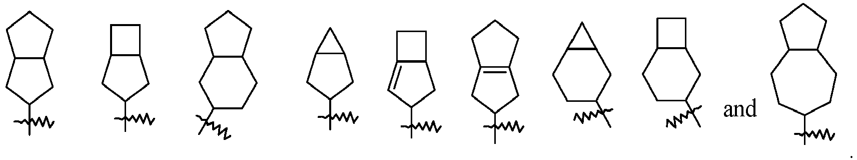

The term "bridged cycloalkyl" refers to a 5 to 20 membered all-carbon polycyclic group, wherein every two rings in the system share two disconnected carbon atoms, wherein the rings can have one or more double bonds, but none of the rings has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include: 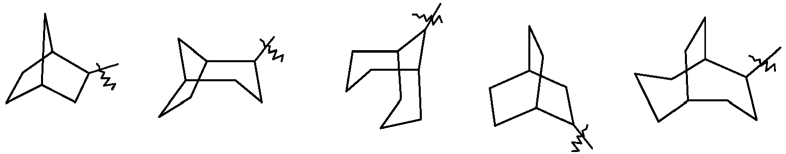 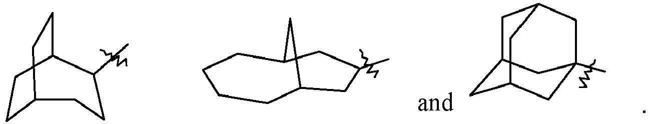 The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is one or more group(s) independently selected from the group consisting of deuterium atom, alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl, carboxylate, -OR¹¹, -C(O)R¹¹, -C(O)OR¹¹, -NR¹²R¹³, -C(O)NR¹²R¹³ and -S(O)ₘR¹¹.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; most preferably, 3 to 8 ring atoms wherein 1 to 3 atoms are heteroatoms; and most preferably 3 to 6 ring atoms wherein 1 to 2 atoms are heteroatoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably piperidinyl, piperazinyl or morpholinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and S(O)ₘ (wherein m is an integer of 0 to 2), with the remaining ring atoms being carbon atoms, where the rings can contain one or more double bonds, but none of the rings has a completely conjugated π-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 3-membered/6-membered, 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

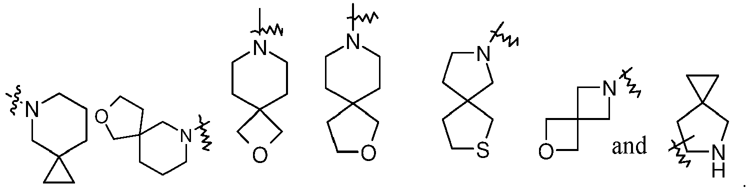

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and the fused heterocyclyl is preferably a bicyclic or tricyclic fused heterocyclyl, and more preferably a 5-membered/5-membered or 5-membered/6-membered bicyclic fused heterocyclyl. Non-limiting examples of fused heterocyclyl include:

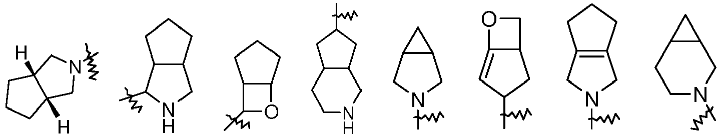

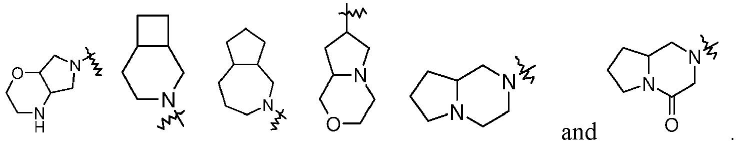

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

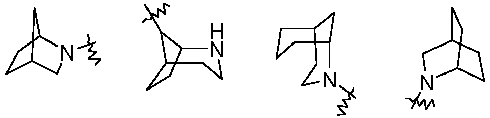

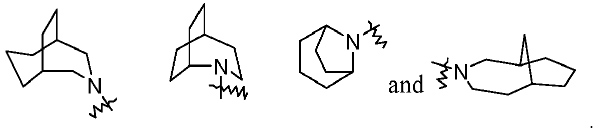

The heterocyclyl ring can be fused to the ring of aryl, heteroaryl or cycloalkyl, wherein the ring bound to the parent structure is heterocyclyl. Non-limiting examples thereof include: 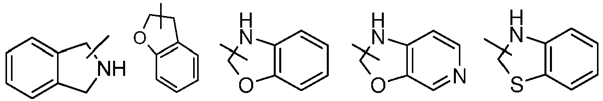 and the like.

The heterocyclyl can be optionally substituted or unsubstituted. When substituted, the substituent group(s) is one or more group(s) independently selected from the group consisting of deuterium atom, alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl, carboxylate, -OR¹¹, -C(O)R¹¹, -C(O)OR¹¹, -NR¹²R¹³, -C(O)NR¹²R¹³ and -S(O)ₘR¹¹.

The term "aryl" refers to a 6 to 14 membered all-carbon monocyclic ring or polycyclic fused ring (i.e. each ring in the system shares an adjacent pair of carbon atoms with another ring in the system) having a conjugated π-electron system, preferably a 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include: 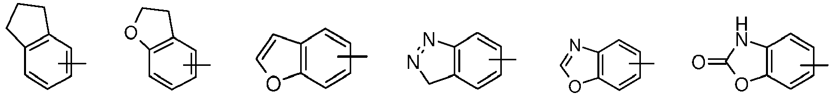 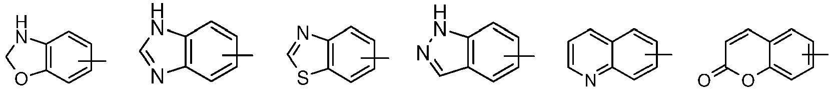 and

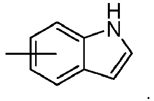

The aryl can be substituted or unsubstituted. When substituted, the substituent group(s) is one or more group(s) independently selected from the group consisting of deuterium atom, alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl, carboxylate, -OR¹¹, -C(O)R¹¹, -C(O)OR¹¹, -NR¹²R¹³, -C(O)NR¹²R¹³ and -S(O)ₘR¹¹.

The term "heteroaryl" refers to a 5 to 14 membered heteroaromatic system having 1 to 4 heteroatoms selected from the group consisting of O, S and N. The heteroaryl is preferably a 5 to 10 membered heteroaryl having 1 to 3 heteroatoms, more preferably a 5 or 6 membered heteroaryl having 1 to 2 heteroatoms; preferably for example, imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, tetrazolyl, pyridyl, thienyl, pyrazolyl, pyrimidinyl, thiazolyl, and more preferably pyridyl. The heteroaryl ring can be fused to the ring of aryl, heterocyclyl or cycloalkyl, wherein the ring bound to the parent structure is heteroaryl ring. Non-limiting examples thereof include:

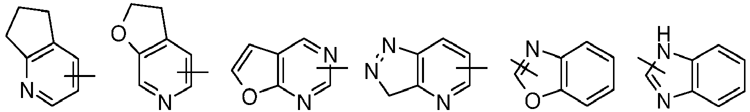

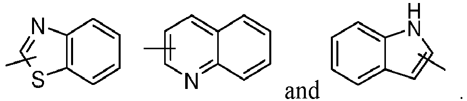

The heteroaryl can be optionally substituted or unsubstituted. When substituted, the substituent group(s) is one or more group(s) independently selected from the group consisting of deuterium atom, alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl, carboxylate, -OR¹¹, -C(O)R¹¹, -C(O)OR¹¹, -NR¹²R¹³, -C(O)NR¹²R¹³ and -S(O)ₘR¹¹.

The term "haloalkyl" refers to an alkyl group substituted by one or more halogen(s), wherein the alkyl is as defined above.

The term "haloalkoxy" refers to an alkoxy group substituted by one or more halogen(s), wherein the alkoxy is as defined above.

The term "hydroxyalkyl" refers to an alkyl group substituted by hydroxy(s), wherein the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "oxo" refers to a =O group.

The term "carbonyl" refers to a C=O group.

The term "carboxyl" refers to a -C(O)OH group.

The term "carboxylate" refers to a -C(O)O(alkyl) or -C(O)O(cycloalkyl) group, wherein the alkyl and cycloalkyl are as defined above.

The term "acyl halide" refers to a compound containing a -C(O)-halogen group.

The present invention also comprises the compounds of formula (I) in various deuterated forms. Each of the available hydrogen atoms attached to the carbon atom can be independently replaced by a deuterium atom. Those skilled in the art can synthesize a compound of formula (I) in a deuterated form with reference to the relevant literature. Commercially available deuterated starting materials can be employed in the preparation of the compound of formula (I) in deuterated form, or they can be synthesized by conventional techniques with deuterated reagents including, but not limited to, deuterated borane, trideuterated borane in tetrahydrofuran, deuterated lithium aluminum hydride, deuterated iodoethane, deuterated iodomethane and the like.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive effort. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present invention, which is safe and effective in mammals and has the desired biological activity.

### Synthesis Method of the Compound of the Present Invention

In order to achieve the object of the present invention, the present invention applies the following technical solutions: 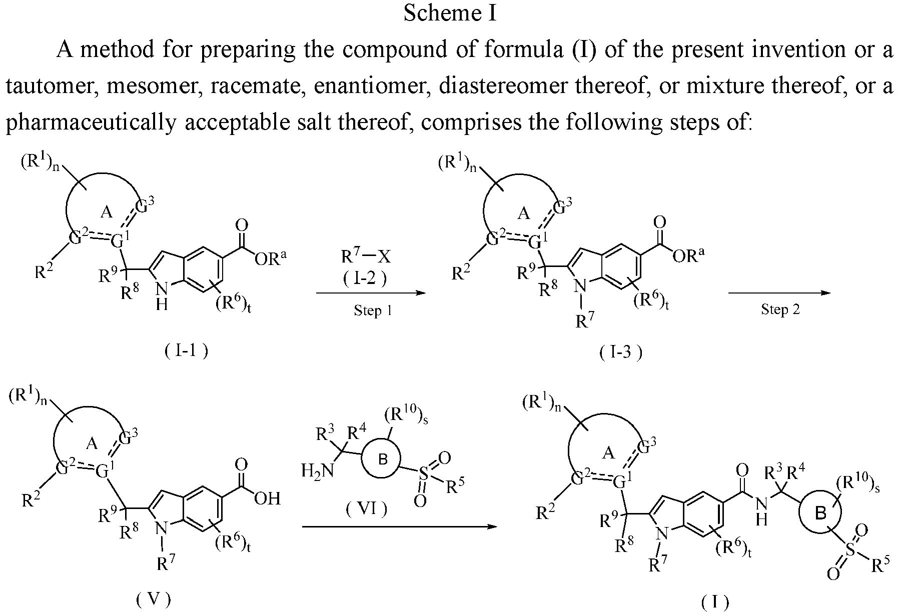 in Step 1, a compound of formula (I-1) and a compound of formula (I-2) are subjected to a nucleophilic substitution reaction under an alkaline condition to obtain a compound of formula (I-3),
in Step 2, the compound of formula (I-3) is hydrolyzed under an alkaline condition to obtain a compound of formula (V),
in Step 3, the compound of formula (V) and a compound of formula (VI) or a pharmaceutically acceptable salt thereof are subjected to a condensation reaction under an alkaline condition in the presence of a condensing agent to obtain the compound of formula (I),
wherein:
X is a halogen;
R^{a} is an alkyl, and preferably methyl or ethyl;
-----, ring A, ring B, G¹∼G³, R¹∼R¹⁰, n, s and t are as defined in formula (I).

The reagent that provides an alkaline condition includes organic bases and inorganic bases. The organic bases include, but are not limited to, triethylamine, *N*,*N*-diisopropylethylamine, *n*-butyllithium, lithium diisopropylamide, potassium acetate, sodium acetate, ammonia, sodium *tert*-butoxide and potassium *tert*-butoxide. The inorganic bases include, but are not limited to, sodium hydride, sodium hydroxide, potassium hydroxide, lithium hydroxide, potassium phosphate, sodium carbonate, sodium bicarbonate, potassium carbonate and cesium carbonate.

The condensing agent includes, but is not limited to, 1-(3-dimethylaminopropyl)-3-ethylcarbodiimide hydrochloride, *N,N'*-dicyclohexylcarbodiimide, *N*,*N*'-diisopropylcarbodiimide, *O*-benzotriazole-*N*,*N*,*N*',*N*'-tetramethyluronium tetrafluoroborate, 1-hydroxybenzotriazole, 1-hydroxy-7-azobenzotriazole, *O*-benzotriazole-*N*,*N*,*N*',*N*'-tetramethyluronium hexafluorophosphate, 2-(7-oxobenzotriazole)-*N*,*N*,*N*',*N*'-tetramethyluronium hexafluorophosphate, 2-(7-azabenzotriazole)-*N*,*N*,*N*',*N*'-tetramethyluronium hexafluorophosphate, benzotriazol-1-yloxytris(dimethylamino)phosphonium hexafluorophosphate and benzotriazol-1-yl-oxytripyrrolidinylphosphonium hexafluorophosphate, and preferably 1-(3-dimethylaminopropyl)-3-ethylcarbodiimide hydrochloride and 1-hydroxybenzotriazole. 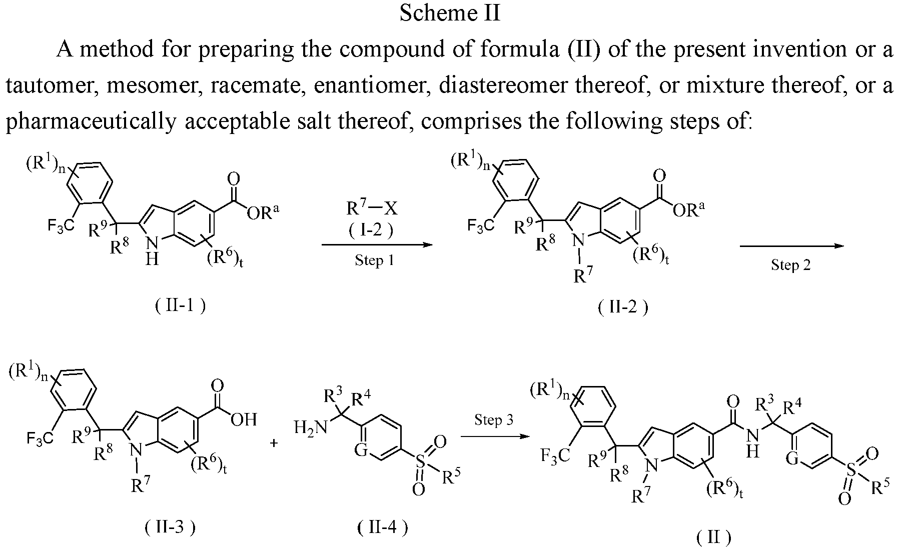 in Step 1, a compound of formula (II-1) and a compound of formula (I-2) are subjected to a nucleophilic substitution reaction under an alkaline condition to obtain a compound of formula (II-2),
in Step 2, the compound of formula (II-2) is hydrolyzed under an alkaline condition to obtain a compound of formula (II-3),
in Step 3, the compound of formula (II-3) and a compound of formula (II-4) or a pharmaceutically acceptable salt thereof are subjected to a condensation reaction under an alkaline condition in the presence of a condensing agent to obtain the compound of formula (II),
wherein:
X is a halogen;
R^{a} is an alkyl, and preferably methyl or ethyl;
G, R¹, R³∼R⁹, n and t are as defined in formula (II).

The reagent that provides an alkaline condition includes organic bases and inorganic bases. The organic bases include, but are not limited to, triethylamine, *N*,*N*-diisopropylethylamine, *n*-butyllithium, lithium diisopropylamide, potassium acetate, sodium acetate, ammonia, sodium *tert*-butoxide and potassium *tert*-butoxide. The inorganic bases include, but are not limited to, sodium hydride, sodium hydroxide, potassium hydroxide, lithium hydroxide, potassium phosphate, sodium carbonate, sodium bicarbonate, potassium carbonate and cesium carbonate.

The condensing agent includes, but is not limited to, 1-(3-dimethylaminopropyl)-3-ethylcarbodiimide hydrochloride, *N,N'*-dicyclohexylcarbodiimide, *N*,*N*'-diisopropylcarbodiimide, *O*-benzotriazole-*N*,*N*,*N*',*N*'-tetramethyluronium tetrafluoroborate, 1-hydroxybenzotriazole, 1-hydroxy-7-azobenzotriazole, *O*-benzotriazole-*N*,*N*,*N*',*N*'-tetramethyluronium hexafluorophosphate, 2-(7-oxobenzotriazole)-*N*,*N*,*N*',*N*'-tetramethyluronium hexafluorophosphate, 2-(7-azabenzotriazole)-*N*,*N*,*N*',*N*'-tetramethyluronium hexafluorophosphate, benzotriazol-1-yloxytris(dimethylamino)phosphonium hexafluorophosphate and benzotriazol-1-yl-oxytripyrrolidinylphosphonium hexafluorophosphate, and preferably 1-(3-dimethylaminopropyl)-3-ethylcarbodiimide hydrochloride and 1-hydroxybenzotriazole. 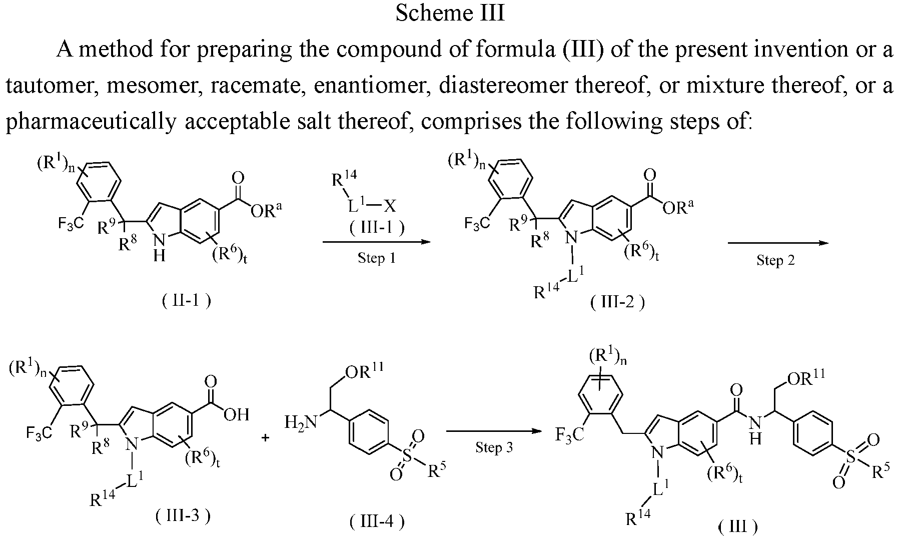 in Step 1, a compound of formula (II-1) and a compound of formula (III-1) are subjected to a nucleophilic substitution reaction under an alkaline condition to obtain a compound of formula (III-2),
in Step 2, the compound of formula (III-2) is hydrolyzed under an alkaline condition to obtain a compound of formula (III-3),
in Step 3, the compound of formula (III-3) and a compound of formula (III-4) or a pharmaceutically acceptable salt thereof are subjected to a condensation reaction under an alkaline condition in the presence of a condensing agent to obtain the compound of formula (III),
wherein:
X is a halogen;
R^{a} is an alkyl, and preferably methyl or ethyl; and
R¹, R⁵, R⁶, R¹¹, R¹⁴, L¹, n and t are as defined in formula (III).

The reagent that provides an alkaline condition includes organic bases and inorganic bases. The organic bases include, but are not limited to, triethylamine, *N*,*N*-diisopropylethylamine, *n*-butyllithium, lithium diisopropylamide, potassium acetate, sodium acetate, ammonia, sodium *tert*-butoxide and potassium *tert*-butoxide. The inorganic bases include, but are not limited to, sodium hydride, sodium hydroxide, potassium hydroxide, lithium hydroxide, potassium phosphate, sodium carbonate, sodium bicarbonate, potassium carbonate and cesium carbonate.

The condensing agent includes, but is not limited to, 1-(3-dimethylaminopropyl)-3-ethylcarbodiimide hydrochloride, *N,N'*-dicyclohexylcarbodiimide, *N*,*N*'-diisopropylcarbodiimide, *O*-benzotriazole-*N*,*N*,*N*',*N*'-tetramethyluronium tetrafluoroborate, 1-hydroxybenzotriazole, 1-hydroxy-7-azobenzotriazole, *O*-benzotriazole-*N*,*N*,*N*',*N*'-tetramethyluronium hexafluorophosphate, 2-(7-oxobenzotriazole)-*N*,*N*,*N*',*N*'-tetramethyluronium hexafluorophosphate, 2-(7-azabenzotriazole)-*N*,*N*,*N*',*N*'-tetramethyluronium hexafluorophosphate, benzotriazol-1-yloxytris(dimethylamino)phosphonium hexafluorophosphate and benzotriazol-1-yl-oxytripyrrolidinylphosphonium hexafluorophosphate, and preferably 1-(3-dimethylaminopropyl)-3-ethylcarbodiimide hydrochloride and 1-hydroxybenzotriazole. 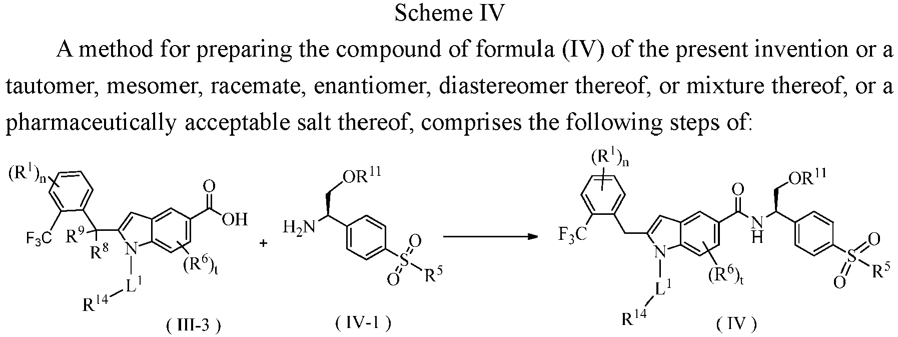 a compound of formula (III-3) and a compound of formula (IV-1) or a pharmaceutically acceptable salt thereof are subjected to a condensation reaction under an alkaline condition in the presence of a condensing agent to obtain the compound of formula (IV),
wherein:
R¹, R⁵, R⁶, R¹¹, R¹⁴, L¹, n and t are as defined in formula (IV).

The reagent that provides an alkaline condition includes organic bases and inorganic bases. The organic bases include, but are not limited to, triethylamine, *N*,*N*-diisopropylethylamine, *n*-butyllithium, lithium diisopropylamide, potassium acetate, sodium acetate, ammonia, sodium *tert*-butoxide and potassium *tert*-butoxide. The inorganic bases include, but are not limited to, sodium hydride, sodium hydroxide, potassium hydroxide, lithium hydroxide, potassium phosphate, sodium carbonate, sodium bicarbonate, potassium carbonate and cesium carbonate.

The condensing agent includes, but is not limited to, 1-(3-dimethylaminopropyl)-3-ethylcarbodiimide hydrochloride, *N,N'*-dicyclohexylcarbodiimide, *N*,*N*'-diisopropylcarbodiimide, *O*-benzotriazole-*N*,*N*,*N*',*N*'-tetramethyluronium tetrafluoroborate, 1-hydroxybenzotriazole, 1-hydroxy-7-azobenzotriazole, *O*-benzotriazole-*N*,*N*,*N*',*N*'-tetramethyluronium hexafluorophosphate, 2-(7-oxobenzotriazole)-*N*,*N*,*N*',*N*'-tetramethyluronium hexafluorophosphate, 2-(7-azabenzotriazole)-*N*,*N*,*N*',*N*'-tetramethyluronium hexafluorophosphate, benzotriazol-1-yloxytris(dimethylamino)phosphonium hexafluorophosphate and benzotriazol-1-yl-oxytripyrrolidinylphosphonium hexafluorophosphate, and preferably 1-(3-dimethylaminopropyl)-3-ethylcarbodiimide hydrochloride and 1-hydroxybenzotriazole.

### DESCRIPTION OF THE DRAWINGS

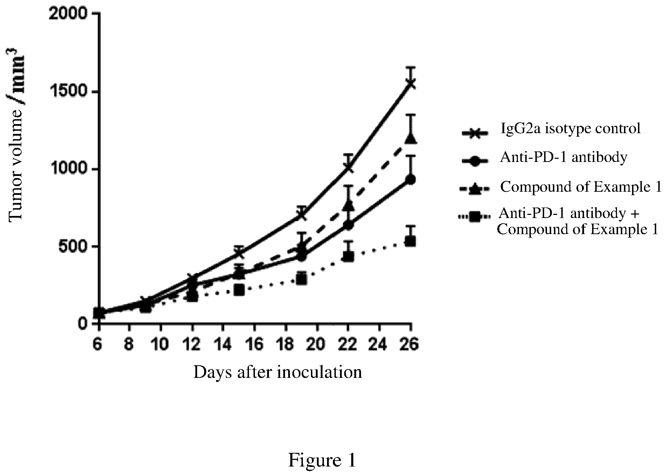

Figure 1 shows the effect of the compound of Example 1 administered alone or in combination with an anti-mouse-PD-1 antibody on MC38 colorectal tumor growth in C57BL/6 mice.

### DETAILED DESCRIPTION OF THE INVENTION

The present invention will be further described with reference to the following examples, but the examples should not be considered as limiting the scope of the present invention.

### EXAMPLES

The structures of the compounds were identified by nuclear magnetic resonance (NMR) and/or mass spectrometry (MS). NMR shifts (δ) are given in 10⁻⁶ (ppm). NMR was determined by a Bruker AVANCE-400 machine. The solvents for determination were deuterated-dimethyl sulfoxide (DMSO-*d₆*), deuterated-chloroform (CDCl₃) and deuterated-methanol (CD₃OD), and the internal standard was tetramethylsilane (TMS).

MS was determined by a SHIMAZU liquid chromatograph-mass spectrometer (manufacturer: Shimazu, type: LC-20AD, LCMS-2020).

High performance liquid chromatography (HPLC) was determined on a Shimadzu SPD-20A high pressure liquid chromatograph (Phenomenex Gemini-NX 5 µM C18 21.2× 100mm chromatographic column), Shimadzu LC-20AD high pressure liquid chromatograph (Phenomenex Luna 3 µM C18 50X2 mm chromatographic column), Agilent HPLC 1200DAD, Agilent HPLC 1200VWD and Waters HPLC e2695-2489 high pressure liquid chromatograph.

Agela MF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm.

ISCO TELEDYNE or AGELA prepacked silica column was generally used for column chromatography.

CombiFlash rapid preparation instrument used was Combiflash Rf200 (TELEDYNE ISCO).

The average kinase inhibition rates and IC₅₀ values were determined by a NovoStar ELISA (BMG Co., Germany).

The known starting materials of the present invention can be prepared by the known methods in the art, or can be purchased from ABCR GmbH & Co. KG, Acros Organnics, Aldrich Chemical Company, Accela ChemBio Inc., Dari chemical Company, or Shanghai Bide Pharmatech Ltd. etc.

Unless otherwise stated, the reactions were carried out under argon atmosphere or nitrogen atmosphere.

"Argon atmosphere" or "nitrogen atmosphere" means that a reaction flask is equipped with an argon or nitrogen balloon (about1 L).

"Hydrogen atmosphere" means that a reaction flask is equipped with a hydrogen balloon (about1 L).

Pressurized hydrogenation reactions were performed on a Parr 3916EKX hydrogenation instrument and a Qinglan QL-500 hydrogen generator or HC2-SS hydrogenation instrument.

In hydrogenation reactions, the reaction system was generally vacuumed and filled with hydrogen, and the above operation was repeated three times.

CEM Discover-S 908860 type microwave reactor was used in microwave reactions.

Unless otherwise stated, the solution refers to an aqueous solution.

Unless otherwise stated, the reaction temperature is room temperature from 20°C to 30°C.

The reaction process in the examples was monitored by thin layer chromatography (TLC). The developing solvent used in the reactions, the eluent system in column chromatography and the developing solvent system in thin layer chromatography for purification of the compounds included: A: dichloromethane/methanol system, B: *n*-hexane/ethyl acetate system, C: *n*-hexane/ethyl acetate/ethanol system, and D: petroleum ether/ethyl acetate system. The ratio of the volume of the solvent was adjusted according to the polarity of the compounds, and a small quantity of alkaline reagent such as triethylamine or acidic reagent such as acetic acid could also be added for adjustment.

### Example 1

### 2-[[4-Chloro-2-(trifluoromethyl)phenyl]methyl]-1-(2,2-dideutero-2-fluoro-ethyl)-N-[(1 R)-1-(4-ethylsulfonylphenyl)-2-hydroxy-ethyl]indole-5-carboxamide 1

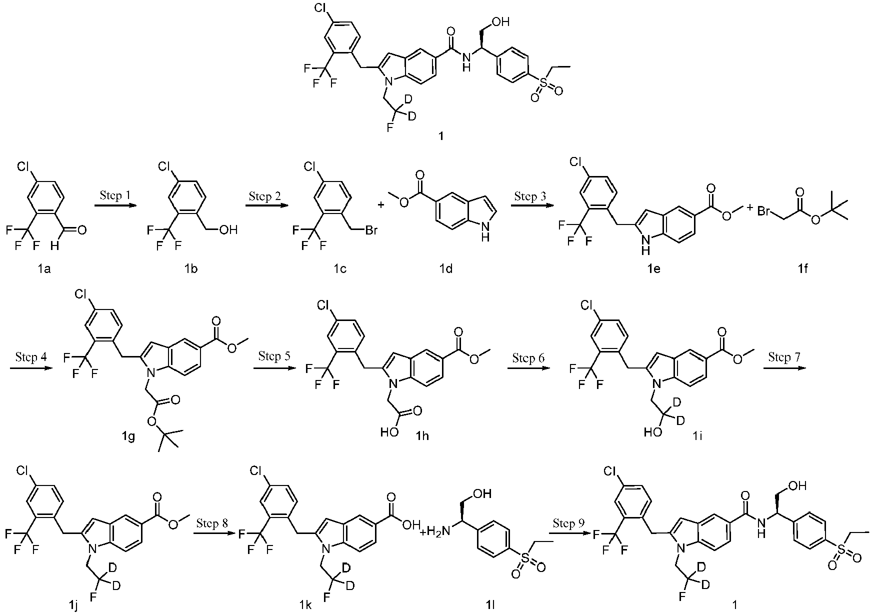

### Step 1

### (4-Chloro-2-(trifluoromethyl)phenyl)methanol 1b

4-Chloro-2-(trifluoromethyl)benzaldehyde **1a** (10 g, 48 mmol, prepared according to the method disclosed in the patent application "WO2011021492") was dissolved in 100 mL of ethanol. To the reaction solution was added sodium borohydride (1.83 g, 48 mmol) in batches. The reaction solution was stirred for 2 hours, and concentrated under reduced pressur. To the resulting residue was added water, and the mixture was extracted with ethyl acetate. The organic phase was washed with saturated sodium chloride solution, dried over anhydrous sodium sulfate, and filtrated. The filtrate was concentrated under reduced pressure, and the resulting residue was purified by silica gel column chromatography with eluent system B to obtain the title compound **1b** (9.5 g, yield: 95%).

### Step 2

### 1-(Bromomethyl)-4-chloro-2-(trifluoromethyl)benzene 1c

Compound **1b** (9.5 g, 45.2 mmol) was dissolved in 100 mL of dichloromethane. To the reaction solution was added dropwise phosphorous tribromide (24.5 g, 90.5 mmol). The reaction solution was stirred for 2 hours, followed by addition of water. The organic phase was separated, and the aqueous phase was extracted with dichloromethane. The organic phases were combined, washed with water, saturated sodium bicarbonate solution and saturated sodium chloride solution successively, dried over anhydrous sodium sulfate, and filtrated. The filtrate was concentrated under reduced pressure to obtain the crude title compound **1c** (10.5 g), which was used directly in the next step without purification.

### Step 3

### Methyl 2-(4-chloro-2-(trifluoromethyl)benzyl)-1H-indole-5-carboxylate 1e

Methyl 1*H*-indole-5-carboxylate **1d** (4.4 g, 25.8 mmol, prepared according to the known method disclosed in *"*Huaxue Shiji, 2015, 37(7), 585-589, 594") was dissolved in 40 mL of *N*,*N*-dimethylacetamide. To the reaction solution were added bis(acetonitrile)palladium dichloride (1.34 g, 5.16 mmol), bicyclo[2.2.1]-2-heptene (4.85 g, 51.6 mmol) and sodium bicarbonate (4.25 g, 51 mmol), followed by the crude compound **1c** (7.4 g, 27 mmol). The reaction solution was warmed up to 70°C and stirred for 12 hours. The reaction solution was cooled to room temperature, followed by addition of 200 mL of water, and extracted with ethyl acetate three times. The organic phases were combined, washed with water and saturated sodium chloride solution successively, dried over anhydrous sodium sulfate, and filtrated. The filtrate was concentrated under reduced pressure, and the resulting residue was purified by silica gel column chromatography with eluent system B to obtain the title compound **1e** (8.1 g, yield: 87%).
MS m/z (ESI): 368.1 [M+1].

### Step 4

### Methyl 1-(2-(tert-butoxy)-2-oxoethyl)-2-(4-chloro-2-(trifluoromethyl)benzyl)-1H-indole-5-carb oxylate 1g

Compound **1e** (21.6 mg, 0.059 mmol), *tert-butyl* 2-bromoacetate **1f** (43.4 µL, 0.294 mmol, prepared according to the known method disclosed in *"*Tetrahedron, 2007, 63(2), 337-346") and cesium carbonate (95.7 mg, 0.294 mmol) were added to 1 mL of *N*,*N*-dimethylformamide. The reaction solution was warmed up to 110°C and stirred for 1.5 hours under microwave. After the reaction was completed, the reaction solution of the title compound **1g** was obtained, which was used directly in the next step without treatment.

### Step 5

### 2-(2-(4-Chloro-2-(trifluoromethyl)benzyl)-5-(methoxycarbonyl)-1H-indol-1-yl)acetic acid 1h

To the above reaction solution of compound **1g** was added 1 mL of methanol and 1 mL of 2*M* potassium hydroxide solution. The reaction solution was stirred for 16 hours. 6*M* hydrochloric acid was added to the reaction solution to adjust the pH to less than 3. The reaction solution was extracted with ethyl acetate three times. The organic phases were combined, washed with saturated sodium chloride solution, dried over anhydrous sodium sulfate, and filtrated. The filtrate was concentrated under reduced pressure, and the resulting residue was purified by high performance liquid chromatography (Shimadzu SPD-20A high pressure liquid chromatograph, Phenomenex Gemini-NX 5 µM C18 21.2×100mm chromatographic column, eluent system: trifluoroacetic acid, water and acetonitrile) to obtain the title compound **1h** (10 mg, yield: 40%).

### Step 6

### Methyl 2-[[4-chloro-2-(trifluoromethyl)phenyl]methyl]-1-(2,2-dideutero-2-hydroxy-ethyl)indol e-5-carboxylate 1i

Compound **1h** (530 mg, 1.24 mmol) was dissolved in 8 mL of tetrahydrofuran. To the reaction solution was added an 1*M* solution (1.52 mL, 1.52 mmol) of trideuterated borane in tetrahydrofuran. The reaction solution was stirred at room temperature for 30 minutes, warmed up to 75°C, and stirred for 45 minutes. After addition of another 0.6 mL of 1*M* solution of trideuterated borane in tetrahydrofuran, the reaction solution was stirred at 75°C for 45 minutes. After addition of 5 mL of methanol, the reaction solution was stirred at 75°C for 10 minutes. The reaction solution was concentrated under reduced pressure, and the resulting residue was purified by silica gel column chromatography with eluent system A to obtain the title compound **1i** (365 mg, yield: 70%).
MS m/z (ESI): 414 [M+1].

### Step 7

### Methyl 2-[[4-chloro-2-(trifluoromethyl)phenyl]methyl]-1-(2,2-dideutero-2-fluoro-ethyl)indole-5-carboxylate 1j

Compound **1i** (365 mg, 0.88 mmol) was dissolved in 10 mL of dichloromethane. The reaction solution was cooled to -78°C in a dry ice-acetone bath, followed by addition of diethylaminosulfur trifluoride (0.23 mL, 1.76 mmol) and stirred for 1 hour. After addition of another diethylaminosulfur trifluoride (0.115 mL, 0.88 mmol) at -78°C, the reaction solution was stirred for 1 hour. The reaction solution was warmed up to 0°C (in an ice-water bath), and stirred for 1 hour. After addition of 20 mL of dichloromethane, the reaction solution was washed with saturated sodium bicarbonate solution, dried over anhydrous sodium sulfate, and filtrated. The filtrate was concentrated under reduced pressure, and the resulting residue was purified by silica gel column chromatography with eluent system A to obtain the title compound **1j** (158 mg, yield: 43%).
MS m/z (ESI): 416 [M+1].

### Step 8

### 2-[[4-Chloro-2-(trifluoromethyl)phenyl]methyl]-1-(2,2-dideutero-2-fluoro-ethyl)indole-5-carboxylic acid 1k

Compound **1j** (158 mg, 0.38 mmol) was dissolved in 16 mL of a mixed solvent of methanol and tetrahydrofuran (V/V = 1:1). After addition of 10 mL of a 2*M* potassium hydroxide solution, the reaction solution was stirred for 16 hours. After addition of another 2 mL of 2*M* potassium hydroxide solution, the reaction solution was stirred for 1 hour. After addition of another potassium hydroxide (0.4 g, 7.13 mmol), the reaction solution was stirred for 2 hours. After addition of another 0.2 g of solid potassium hydroxide, the reaction solution was stirred at room temperature for 1 hour. 6*M* hydrochloric acid was added dropwise to the reaction solution until the pH was less than 2, then 50 mL of water was added. The reaction solution was extracted with ethyl acetate (30 mL×3). The organic phases were combined, washed with saturated sodium chloride solution, dried over anhydrous sodium sulfate, and filtrated. The filtrate was concentrated under reduced pressure to obtain the crude title compound **1k** (160 mg), which was used directly in the next step without purification.
MS m/z (ESI): 402 [M+1].

### Step 9

### 2-[[4-Chloro-2-(trifluoromethyl)phenyl]methyl]-1-(2,2-dideutero-2-fluoro-ethyl)-N-[(1 R)-1-(4-ethylsulfonylphenyl)-2-hydroxy-ethyl]indole-5-carboxamide 1

The crude compound **1k** (160 mg, 0.4 mmol) and (*R*)-2-amino-2-(4-(ethylsulfonyl)phenyl)ethanol **1l** (211 mg, 0.8 mmol, prepared according to the method disclosed in the patent application "US9481674") were dissolved in 50 mL of dichloromethane. To the reaction solution were added 1-(3-dimethylaminopropyl)-3-ethylcarbodiimide hydrochloride (153 mg, 0.8 mmol), 1-hydroxybenzotriazole (108 mg, 0.8 mmol) and triethylamine (280 µL, 2.0 mmol). The reaction solution was stirred for 48 hours, followed by addition of 20 mL of dichloromethane, washed with 20 mL of water, dried over anhydrous sodium sulfate, and filtrated. The filtrate was concentrated under reduced pressure, and the resulting residue was purified by silica gel column chromatography with eluent system C to obtain the title compound **1** (180 mg, yield: 70%).
MS m/z (ESI): 613 [M+1].
¹H NMR (500 MHz, CDCl₃) δ 8.08 (s, 1H), 7.91-7.85 (d, 2H), 7.77-7.64 (m, 2H), 7.59 (d, 2H), 7.42 (dd, 1H), 7.32 (d, 1H), 7.12-7.10 (d, 1H), 7.09-7.07 (d, 1H), 6.29 (s, 1H), 5.33 (dt, 1H), 5.30 (s, 1H), 4.31 (s, 3H), 4.25 (s, 1H), 4.12-3.97 (m, 2H), 3.09 (q, 2H), 1.28 (t, 3H).

### Example 2

### 1-Cyclopropyl-2-[dideutero-[5-(trifluoromethyl)pyrazol-1-yl]methyl]-N-[(1R)-1-(4-ethy lsulfonylphenyl)-2-hydroxy-ethyl]indole-5-carboxamide 2

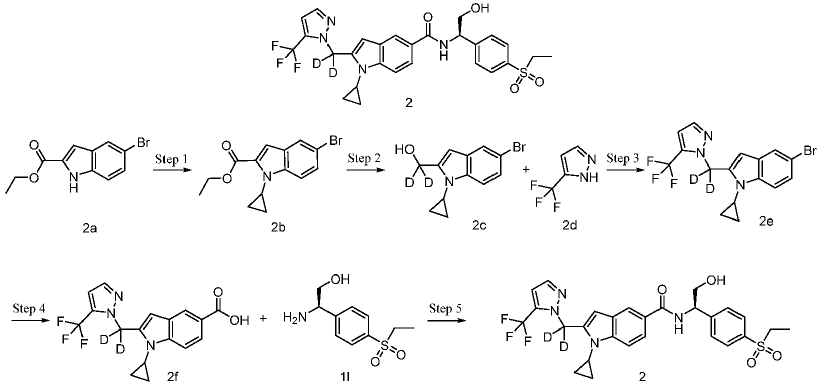

### Step 1

### Ethyl 5-bromo-1-cyclopropyl-1H-indole-2-carboxylate 2b

Ethyl 5-bromo-1*H*-indole-2-carboxylate **2a** (0.76 g, 2.84 mmol, prepared according to the method disclosed in the patent application "WO2014060386") and cyclopropylboronic acid (1.22 g, 14.2 mmol, prepared according to the method disclosed in the patent application "WO2008024843") were dissolved in 8 mL of 1,2-dichloroethane. To the reaction solution were added copper acetate (1.13 g, 5.96 mmol), 2,2'-bipyridine (0.98 g, 6.25 mmol) and sodium carbonate (0.66 g, 6.25 mmol). The reaction solution was stirred at 80°C for 16 hours, and filtrated. The filter cake was washed with dichloromethane. The filtrates were combined and concentrated under reduced pressure. The resulting residue was purified by silica gel column chromatography with eluent system B to obtain the title compound **2b** (920 mg, yield: 100%).
MS m/z (ESI): 308 [M+1]

### Step 2

### (5-Bromo-1-cyclopropyl-indol-2-yl)-dideutero-methanol 2c

Deuterated lithium aluminum hydride (66 mg, 1.57 mmol) was suspended in 2 mL of tetrahydrofuran. The reaction solution was cooled to 0°C, followed by dropwise addition of 1 mL of a pre-prepared solution of compound **2b** (0.25 g, 0.81 mmol) in tetrahydrofuran, and stirred at 0°C for 2 hours. To the reaction solution was added a small amount of a mixed solvent of methanol and water (V:V = 1:1), and the resulting suspension was filtrated through celite. The filtrate was purified by CombiFlash rapid preparation instrument with eluent system B to obtain the title compound **2c** (86 mg, yield: 39.6%).
MS m/z (ESI): 268 [M+1].

### Step 3

### 5-Bromo-1-cyclopropyl-2-[dideutero-[5-(trifluoromethyl)pyrazol-1-yl]methyl]indole 2e

Compound **2c** (80 mg, 0.3 mmol) and 5-(trifluoromethyl)-1*H*-pyrazole **2d** (81 mg, 0.54 mmol, prepared according to the known method disclosed in *"*Journal of Heterocyclic Chemistry, 2010, 47(2), 301-308") were dissolved in 3 mL of tetrahydrofuran. To the reaction solution were added triphenylphosphine (142 mg, 0.54 mmol) and diethylazodicarboxylate (94 mg, 0.54 mmol). The reaction solution was stirred for 16 hours, followed by addition of 30 mL of ethyl acetate, washed with water (10 mL) and saturated sodium chloride solution (10 mL) successively, dried over anhydrous sodium sulfate, and filtrated. The filtrate was concentrated under reduced pressure, and the resulting residue was purified by silica gel column chromatography with eluent system B to obtain the title compound **2e** (30 mg, yield: 25.97%).
MS m/z (ESI):386 [M+1].

### Step 4

### 1-Cyclopropyl-2-[dideutero-[5-(trifluoromethyl)pyrazol-1-yl]methyl]indole-5-carboxyli c acid 2f

Compound **2e** (30 mg, 0.078 mmol), molybdenum hexacarbonyl (40 mg, 0.15 mmol), *trans*-di-(*M*)-bis[2-(di-*o*-tolylphosphine)benzyl]dipalladium (II) acetate (20 mg, 0.02 mmol), tri-*tert*-butylphosphine tetrafluoroborate (20 mg, 0.069 mmol) and 1,8-diazabicyclo[5.4.0]undec-7-ene (50 µL, 0.34 µmol) were added to a mixed solvent of water (50 µL) and 1,4-dioxane (0.6 mL). The reaction solution was stirred for 15 minutes at 150°C under microwave. The reaction solution was purified by CombiFlash rapid preparation instrument with eluent system A to obtain the title compound **2f** (13 mg, yield: 47.5%).
MS m/z (ESI): 352 [M+1].

### Step 5

### 1-Cyclopropyl-2-[dideutero-[5-(trifluoromethyl)pyrazol-1-yl]methyl]-N-[(1R)-1-(4-ethy lsulfonylphenyl)-2-hydroxy-ethyl]indole-5-carboxamide 2

Compound **2f** (7 mg, 0.02 mmol) was dissolved in 0.8 mL of *N*,*N*-dimethylformamide. To the reaction solution were added compound **1l** (7 mg, 0.03 mmol), *N*,*N*-diisopropylethylamine (20 µL, 0.12 µmmol) and 2-(7-azabenzotriazole)-*N*,*N*,*N*',*N*'-tetramethyluronium hexafluorophosphate (15 mg, 0.095 mmol). After stirring for 2 hours, the reaction solution was purified by high performance liquid chromatography (Shimadzu SPD-20A high pressure liquid chromatograph, Phenomenex Gemini-NX 5 µM C18 21.2×100mm chromatographic column, eluent system: trifluoroacetic acid, water and acetonitrile) to obtain the title compound **2** (3 mg, yield: 26.7%).
MS m/z (ESI): 563 [M+1].
¹H NMR (500MHz, CDCl₃) δ 7.95 (d, 1H), 7.82 (d, 2H), 7.64 (dd, 1H), 7.61-7.49 (m, 4H), 7.02 (d, 1H), 6.64 (d, 1H), 6.22 (s, 1H), 5.28-5.23 (m, 1H), 4.02 (dd, 1H), 3.95 (dd, 1H), 3.16 (tt, 1H), 3.06-3.00 (m, 3H), 1.26-1.12 (m, 5H), 1.08-0.96 (m, 2H).

### Example 3

### 2-[[4-Chloro-2-(trifluoromethyl)phenyl]methyl]-N-[dideutero-(4-ethylsulfonylphenyl)m ethyl]-1-(2-fluoroethyl)indole-5-carboxamide 3

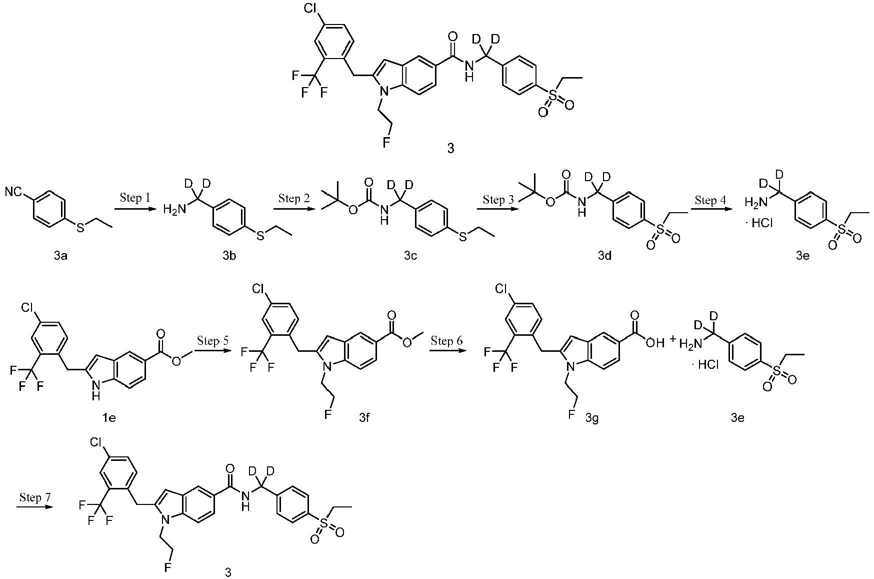

### Step 1

Dideutero-(4-ethylthiophenyl)methanamine **3b** 4-(Ethylthio)benzonitrile **3a** (1.36 g, 8.34 mmol, prepared according to the method disclosed in the patent application "lo-(4-") was dissolved in 50 mL of tetrahydrofuran, followed by addition of deuterated lithium aluminum hydride (0.668 g, 17 mmol) in batches. The reaction solution was stirred for 0.5 hour. A small amount of water was added to quench the reaction. Another 50 mL of water was added, and the reaction solution was filtrated. The filter cake was washed with 50 mL of ethyl acetate. The filtrate was separated, and the aqueous phase was extracted with ethyl acetate (20 mL×2). The organic phases were combined, washed with saturated sodium chloride solution (20 mL), dried over anhydrous magnesium sulfate, and filtrated. The filtrate was concentrated under reduced pressure to obtain the crude title compound **3b** (1.47 g), which was used directly in the next step without purification.

### Step 2

### Tert-butyl N-[dideutero-(4-ethylthiophenyl)methyl]carbamate 3c

The crude compound **3b** (1.47 g, 8.68 mmol) was dissolved in 100 mL of dichloromethane. To the reaction solution was added di-*tert*-butyl dicarbonate (2.84 g, 13.03 mmol). The reaction solution was stirred for 0.5 hour, washed with water (20 mL), dried over anhydrous sodium sulfate, and filtrated to obtain a solution of the title compound **3c**, which was used directly in the next step without treatment.

### Step 3

### Tert-butyl N-[dideutero-(4-ethylsulfonylphenyl)methyl]carbamate 3d

To the above solution comprising compound **3c** was added *m*-chloroperoxybenzoic acid (7.02 g, 43.4 mmol). The reaction solution was stirred for 16 hours, washed with saturated sodium thiosulfate solution (30 mL×2) and saturated sodium bicarbonate solution (30 mL) successively, dried over anhydrous sodium sulfate, and filtrated. The filtrate was concentrated under reduced pressure, and the resulting residue was purified by silica gel column chromatography with eluent system B to obtain the title compound **3d** (1.97 g, yield: 75.5%).

### Step 4

Dideutero-(4-ethylsulfonylphenyl)methanamine hydrochloride **3e** Compound **3d** (1.97 g, 6.53 mmol) was dissolved in 15 mL of dichloromethane. To the reaction solution was added a 2*M* solution of hydrogen chloride in ether (20 mL, 40 mmol). The reaction solution was stirred for 2 hours, followed by addition of another 2*M* solution of hydrogen chloride in ether (15 mL, 30 mmol), and stirred for 16 hours. The reaction solution was filtrated, and the filter cake was collected to obtain the crude title compound **3e** (1.465 g), which was used directly in the next step without purification.

### Step 5

### Methyl 2-(4-chloro-2-(trifluoromethyl)benzyl)-1-(2-fluoroethyl)-1H-indole-5-carboxylate 3f

Compound **1e** (0.3 g, 815.77 µmol) and 1-bromo-2-fluoroethane (310.7 mg, 2.45 mmol) were dissolved in 10 mL of *N*,*N*-dimethylformamide. To the reaction solution was added cesium carbonate (797.38 mg, 2.45 mmol). The reaction solution was stirred for 1 hour at 100°C under microwave, cooled and filtrated. The filtrate was concentrated under reduced pressure, and the resulting residue was purified by silica gel column chromatography with eluent system B to obtain the title compound **3f** (0.25 g, yield: 74.06%).
MS m/z (ESI): 414.1 [M+1].

### Step 6

### 2-(4-Chloro-2-(trifluoromethyl)benzyl)-1-(2-fluoroethyl)-1H-indole-5-carboxylic acid 3g

Compound **3f** (0.25 g, 604.17 µmol) was dissolved in 20 mL of methanol. To the reaction solution was added 1.5 mL of 4*M* sodium hydroxide solution. The reaction solution was stirred under reflux for 1 hour, and then cooled to room temperature. 1*M* hydrochloric acid was added dropwise to adjust the pH to 3∼4. After addition of 20 mL of water and 20 mL of ethyl acetate, the reaction solution was extracted with ethyl acetate (20 mL). The organic phases were combined, dried over anhydrous sodium sulfate, and filtrated. The filtrate was concentrated under reduced pressure, and the resulting residue was purified by silica gel column chromatography with eluent system A to obtain the title compound **3g** (0.24 g, yield: 99.4%).
MS m/z (ESI): 400.1 [M+1].

### Step 7

### 2-[[4-Chloro-2-(trifluoromethyl)phenyl]methyl]-N-[dideutero-(4-ethylsulfonylphenyl)m ethyl]-1-(2-fluoroethyl)indole-5-carboxamide 3

Compound **3g** (10 mg, 25.01 µmol) was dissolved in 1 mL of *N*,*N*-dimethylformamide. To the reaction solution were added the crude compound **3e** (25 mg, 0.124 mmol) and triethylamine (28 µL, 0.2 mmol), followed by 1-(3-dimethylaminopropyl)-3-ethylcarbodiimide hydrochloride (12 mg, 0.0625 mmol) and 1-hydroxybenzotriazole (8.5 mg, 0.0625 mmol). The reaction solution was stirred for 16 hours, and concentrated under reduced pressure. The the resulting residue was purified by high performance liquid chromatography (Shimadzu SPD-20A high pressure liquid chromatograph, Phenomenex Gemini-NX 5 µM C18 21.2×100mm chromatographic column, eluent system: trifluoroacetic acid, water and acetonitrile) to obtain the title compound **3** (2.6 mg, yield: 17.8%).
MS m/z (ESI): 583 [M+1].

### Example 4

### 1-Cyclopropyl-N-[dideutero-(4-ethylsulfonylphenyl)methyl]-2-[[2-(trifluoromethyl)phe nyl]methyl]indole-5-carboxamide 4

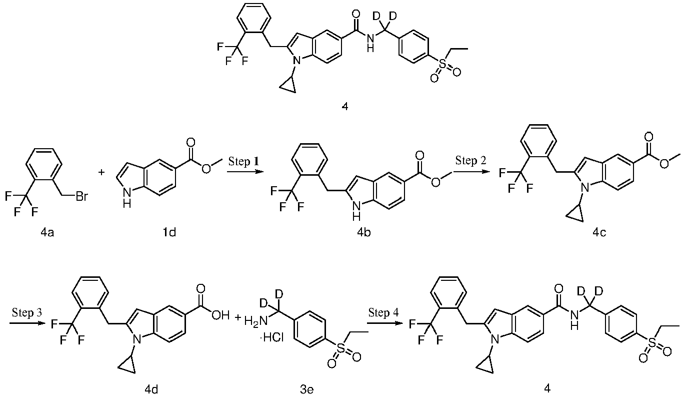

### Step 1

Methyl 2-(2-(trifluoromethyl)benzyl)-1*H*-indole-5-carboxylate **4b** Compound **1d** (400 mg, 2.29 mmol), 1-(bromomethyl)-2-(trifluoromethyl)benzene **4a** (574 mg, 2.4 mmol, prepared according to the method disclosed in the patent application " WO2015176640"), bis(acetonitrile)palladium (II) dichloride (118 mg, 0.46 mmol), bicyclo[2.2.1]-2-heptene (429 mg, 4.6 mmol) and sodium bicarbonate (384 mg, 4.6 mmol) were added to 10 mL of *N*,*N*-dimethylacetamide. The reaction solution was heated to 70°C and stirred for 16 hours under an argon atmosphere. The reaction solution was cooled to room temperature, poured into water, and extracted with ethyl acetate three times. The organic phases were combined, washed with water and saturated sodium chloride solution successively, dried over anhydrous sodium sulfate, and filtrated. The filtrate was concentrated under reduced pressure, and the resulting residue was purified by silica gel column chromatography with eluent system B to obtain the title compound **4b** (570 mg, yield: 74.9%).

### Step 2

### Methyl 1-cyclopropyl-2-(2-(trifluoromethyl)benzyl)-1H-indole-5-carboxylate 4c

Compound **4b** (64 mg, 192 µmol), cyclopropylboronic acid (100 mg, 1150 µmol), 2,2'-bipyridine (63 mg, 404 µmol), copper acetate (73 mg, 404 µmol) and sodium carbonate (43 mg, 404 µmol) were added to 5 mL of tetrahydrofuran. The reaction solution was stirred at 60°C for 16 hours under an argon atmosphere. The reaction solution was filtrated, and the filtrate was extracted with ethyl acetate, dried over anhydrous sodium sulfate and filtrated. The filtrate was concentrated under reduced pressure, and the resulting residue was purified by silica gel column chromatography with eluent system B to obtain the title compound **4c** (70 mg, yield: 98%).
MS m/z (ESI): 374 [M+1].

### Step 3

### 1-Cyclopropyl-2-(2-(trifluoromethyl)benzyl)-1H-indole-5-carboxylic acid 4d

Compound **4c** (70 mg, 188 µmol) and 2*M* potassium hydroxide solution (1.5 mL, 3.0 mmol) were added to 1.5 mL of methanol, and the reaction solution was stirred for 16 hours. The reaction solution was concentrated under reduced pressure to remove methanol. To the resulting residue was added dropwise 1*M* hydrochloric acid to adjust the pH to 3, and the mixture was extracted with ethyl acetate. The organic phases were combined, dried over anhydrous sodium sulfate, and filtrated. The filtrate was concentrated under reduced pressure to obtain the crude title compound **4d** (70 mg), which was used directly in the next step without purification.
MS m/z (ESI):360 [M+1].

### Step 4

### 1-Cyclopropyl-N-[dideutero-(4-ethylsulfonylphenyl)methyl]-2-[[2-(trifluoromethyl)phe nyl]methyl]indole-5-carboxamide 4

In accordance with the synthetic route in Step 7 of Example 3, the starting compound **3g** was replaced with the crude compound **4d**, accordingly, the title compound **4** (1.0 mg) was prepared.
MS m/z (ESI):543 [M+1].

### Example 5

### 2-[[4-Chloro-2-(trifluoromethyl)phenyl]methyl]-1-(2,2-dideutero-2-hydroxy-ethyl)-N-[( 1R)-1-(4-ethylsulfonylphenyl)-2-hydroxy-ethyl]indole-5-carboxamide 5

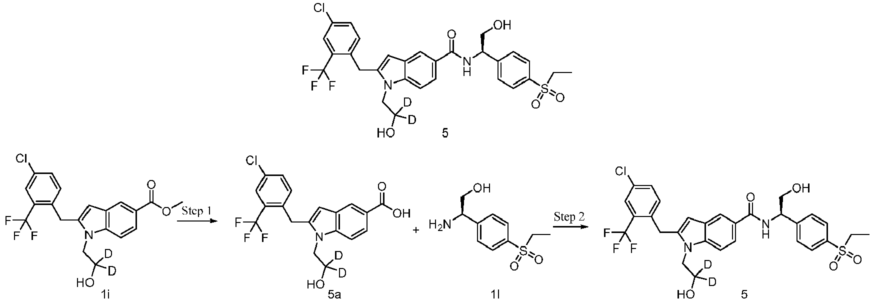

### Step 1

### 2-[[4-Chloro-2-(trifluoromethyl)phenyl]methyl]-1-(2,2-dideutero-2-hydroxy-ethyl)indol e-5-carboxylic acid 5a

In accordance with the synthetic route in Step 8 of Example 1, the starting compound **1j** was replaced with compound **1i**, accordingly, the title compound **5a** (3.3 mg) was prepared.
MS m/z (ESI): 400 [M+1].

### Step 2

### 2-[[4-Chloro-2-(trifluoromethyl)phenyl]methyl]-1-(2,2-dideutero-2-hydroxy-ethyl)-N-[( 1R)-1-(4-ethylsulfonylphenyl)-2-hydroxy-ethyl]indole-5-carboxamide 5

In accordance with the synthetic route in Step 9 of Example 1, the starting compound **1k** was replaced with compound **5a**, accordingly, the title compound **5** (2.0 mg) was prepared.
MS m/z (ESI): 611 [M+1].
¹H NMR (500 MHz, CDCl₃) δ 8.06 (d, 1H), 7.92-7.86 (m, 2H), 7.71 (d, 1H), 7.69 (dd, 1H), 7.63-7.57 (m, 2H), 7.41 (dd, 1H), 7.39 (d, 1H), 7.11 (d, 1H), 7.09-7.05 (m, 1H), 6.27-6.23 (m, 1H), 5.34 (dt, 1H), 4.36 (s, 2H), 4.16 (s, 2H), 4.13-3.99 (m, 2H), 3.10 (q, 2H), 1.29 (t, 3H).

### Example 6

### 2-[[4-Chloro-2-(trifluoromethyl)phenyl]methyl]-1-(2-fluoroethyl)-N-[(1R)-2-hydroxy-1 -[4-(1,1,2,2,2-pentadeuteroethylsulfonyl)phenyl]ethyl]indole-5-carboxamide 6

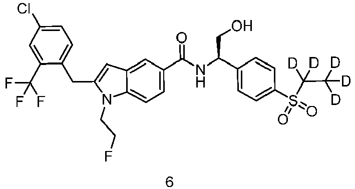

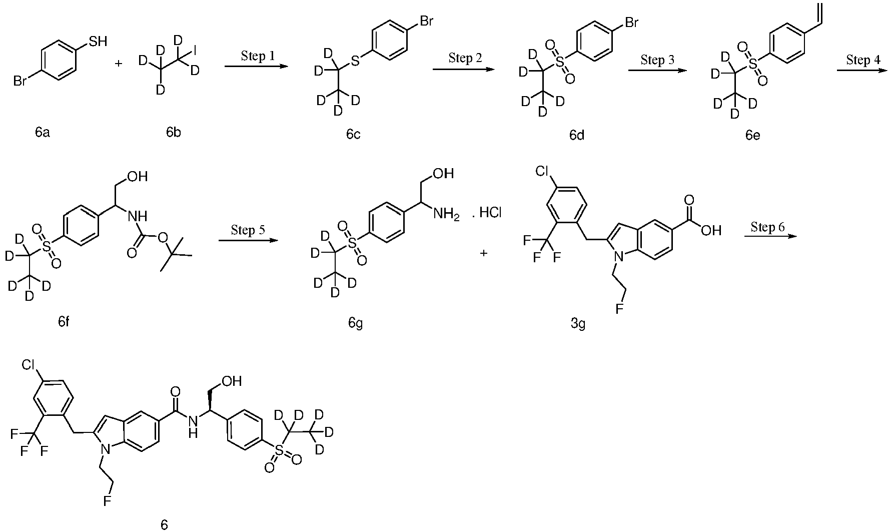

### Step 1

### 1-Bromo-4-(1,1,2,2,2-pentadeuteroethylthio)benzene 6c

4-bromobenzenethiol **6a** (1 g, 5.29 mmol, Adamas), 1,1,1,2,2-pentadeutero-2-iodo-ethane **6b** (1.1 g, 6.35 mmol, Sigma) and cesium carbonate (5.25 g, 15.87 mmol) were added to 50 mL of *N*,*N*-dimethylformamide, and the reaction solution was stirred for 16 hours. The reaction solution was filtrated, and the filtrate was concentrated under reduced pressure. The resulting residue was purified by silica gel column chromatography with eluent system D to obtain the title compound **6c** (1.1 g, yield: 93.62%).

### Step 2

### 1-Bromo-4-(1,1,2,2,2-pentadeuteroethylsulfonyl)benzene 6d

Compound **6c** (1.17 g, 4.95 mmol) was dissolved in 20 mL of methanol. To the reaction solution was added 5 mL of a pre-prepared solution of potassium peroxymonosulfate (6.1 g, 9.90 mmol). The reaction solution was stirred for 2 hours, and filtrated. The filtrate was concentrated under reduced pressure, and the resulting residue was purified by silica gel column chromatography with eluent system D to obtain the title compound **6d** (0.9 g, yield: 71.52%).
MS m/z (ESI): 271 [M+18].

### Step 3

### 1-(1,1,2,2,2-Pentadeuteroethylsulfonyl)-4-vinyl-benzene 6e

Compound **6d** (0.8 g, 3.46 mmol) and 4,4,5,5-tetramethyl-2-vinyl-1,3,2-dioxaborolane (1 g, 6.92 mmol, Accela ChemBio (Shanghai) Inc) were dissolved in 12 mL of a mixed solvent of 1,4-dioxane and water (V:V = 5:1). To the reaction solution were added tetratriphenylphosphine palladium (400 mg, 346.24 µmol) and cesium carbonate (2.3 g, 6.92 mmol). The reaction system was stirred under an argon atmosphere at 95°C for 18 hours. The reaction solution was cooled to room temperature, and extracted with ethyl acetate (30 mL×2). The organic phases were combined, dried over anhydrous sodium sulfate, and filtrated. The filtrate was concentrated under reduced pressure, and the resulting residue was purified by silica gel column chromatography with eluent system D to obtain the title compound **6e** (0.6 g, yield: 86.09%).
MS m/z (ESI): 219 [M+18].

### Step 4

### Tert-butyl N-[2-hydroxy-1-[4-(1,1,2,2,2-pentadeuteroethylsulfonyl)phenyl]ethyl]carbamate 6f

Sodium hydroxide (410 mg, 10.28 mmol) was dissolved in water (20 mL), and 5 mL of the resulting solution was taken to dissolve potassium osmate dihydrate (60 mg, 137.11 µmol). 15 mL of a pre-prepared solution of *tert-butyl* carbamate (98%, 1.5 g, 12.00 mmol) in *n*-propanol was added to the above sodium hydroxide solution. The reaction solution was cooled in an ice-water bath, followed by dropwise addition of *tert*-butyl hypochlorite (1.2 g, 10.28 mmol), and stirred for 5 minutes. To the reaction solution were added 5 mL of a pre-prepared solution of hydroquinidine 1,4-phthalazinediyl ether (0.17 g, 205.67 µmol) in *n*-propanol, compound **6e** (0.6 g, 3.43 mmol) and the above potassium osmate dihydrate solution. The reaction system was stirred at 25°C for 1 hour. The reaction solution was concentrated under reduced pressure, followed by addition of 20 mL of water, and extracted with ethyl acetate (30 mL×2). The organic phases were combined, dried over anhydrous sodium sulfate, and filtrated. The filtrate was concentrated under reduced pressure, and the resulting residue was purified by silica gel column chromatography with eluent system D to obtain the title compound **6f** (R/S=14/1) (0.5 g, yield: 43.62%).
MS m/z (ESI): 235 [M+1-100], 279 [M+1-56].

### Step 5

### 2-Amino-2-[4-(1,1,2,2,2-pentadeuteroethylsulfonyl)phenyl]ethanol hydrochloride 6g

Compound **6f** (0.5 g, 1.5 mmol) was dissolved in 15 mL of methanol. To the reaction solution was added 3 mL of concentrated hydrochloric acid. The reaction solution was stirred for 1 hour, and then concentrated under reduced pressure to obtain the crude title compound **6g** (R/S=14/1) (350 mg, yield: 86.46%).
MS m/z (ESI): 235 [M+1].

### Step 6

### 2-[[4-Chloro-2-(trifluoromethyl)phenyl]methyl]-1-(2-fluoroethyl)-N-[(1R)-2-hydroxy-1 -[4-(1,1,2,2,2-pentadeuteroethylsulfonyl)phenyl]ethyl]indole-5-carboxamide 6

Compound **3g** (0.15 g, 500.29 µmol) and the crude compound **6g** (150 mg, 650.38 µmol) were dissolved in 5 mL of *N*,*N*-dimethylformamide. To the reaction solution were added 2-(7-oxobenzotriazole)-*N*,*N*,*N*',*N*'-tetramethyluronium hexafluorophosphate (0.4 g, 1 mmol) and *N*,*N*-diisopropylethylamine (0.2 g, 1 mmol). The reaction solution was stirred for 1 hour, and then concentrated under reduced pressure. The resulting residue was purified by high performance liquid chromatography (Waters 2767-SQ high pressure liquid chromatograph, sunfire OBD, 150^{∗}19 mm 5nfi chromatographic column, eluent system: ammonium bicarbonate, water and acetonitrile) to obtain the crude title compound **6** (70 mg). The crude product was separated chirally (separation conditions: chiral preparative column Amylose-1 20^{∗}250mm, 5 µm; mobile phase: ethanol/n-hexane = 40/60 (v/v), flow rate: 20 mL/minute). The corresponding fractions were collected and concentrated under reduced pressure to obtain the title compound **6** (55.8 mg, yield: 18.10%).
MS m/z (ESI): 616 [M+1].

Chiral HPLC: retention time 13.798 minutes (chromatographic column: Lux Amylose-1 (AD) 4.6^{∗}150 mm 5 µm; mobile phase: ethanol (containing 0.1% of diethylamine)/*n*-hexane = 40/60 (v/v)).
¹HNMR(400MHz, CDCl₃) δ 8.11 (s, 1H), 7.88-7.86 (d, 2H), 7.75-7.72 (m, 2H), 7.61-7.59 (d, 2H), 7.47-7.44 (d, 1H), 7.34-7.32 (m, 1H), 7.29-7.27 (m, 1H), 7.13-7.11 (d, 1H), 6.30 (s, 1H), 5.33-5.32 (m, 1H), 4.69-4.66 (m, 1H), 4.57-4.55 (m, 1H), 4.34 (s, 3H), 4.30-4.27 (m, 1H), 4.08-4.05 (dd, 1H), 4.01-3.97 (dd, 1H).

### Example 7

### 2-[[4-Chloro-2-(trifluoromethyl)phenyl]methyl]-1-(2-fluoroethyl)-N-[(1R)-2-hydroxy-1 -[4-(trideuteromethylsulfonyl)phenyl]ethyl]indole-5-carboxamide

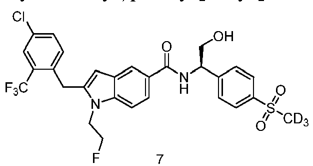

In accordance with the synthetic route in Example 6, the starting compound **6b** was replaced with deuterated iodomethane, accordingly, the title compound **7** was prepared.

### Example 8

### 2-[[4-Chloro-2-(trifluoromethyl)phenyl]methyl]-N-[(1R)-1-(4-ethylsulfonylphenyl)-2-(t rideuteromethoxy)ethyl]-1-(2-fluoroethyl)indole-5-carboxamide 8

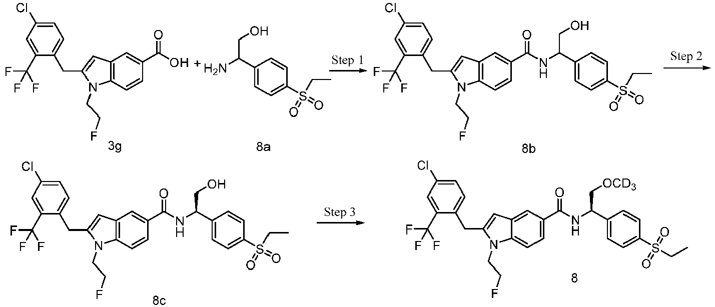

### Step 1

### 2-(4-Chloro-2-(trifluoromethyl)benzyl)-N-(1-(4-(ethylsulfonyl)phenyl)-2-hydroxyethyl) -1-(2-fluoroethyl)-1H-indole-5-carboxamide 8b

Compound **3g** (10 mg, 25.01 µmol) was dissolved in 2 mL of *N*,*N*-dimethylformamide. To the reaction solution were added compound 2-amino-2-(4-ethylsulfonylphenyl)ethanol **8a** (8.67 mg, 37.83 µmol) and *N*,*N*-diisopropylethylamine (6.47 mg, 50.03 µmol), followed by 2-(7-azabenzotriazole)-*N*,*N*,*N*',*N*'-tetramethyluronium hexafluorophosphate (11.77 mg, 50.03 µmol). The reaction solution was stirred at room temperature for 2 hours, and then concentrated under reduced pressure. The resulting residue was purified by high performance liquid chromatography (Waters 2767-SQ high pressure liquid chromatograph, sunfire OBD, 150^{∗}19 mm 5nfi chromatographic column, eluent system: ammonium bicarbonate, water and acetonitrile) to obtain the title compound **8b** (7.9 mg, 51.7%).
MS m/z (ESI): 611.5 [M+1].

### Step 2

### (R)-2-(4-Chloro-2-(trifluoromethyl)benzyl)-N-(1-(4-(ethylsulfonyl)phenyl)-2-hydroxyet hyl)-1-(2-fluoroethyl)-1H-indole-5-carboxamide 8c

Compound **8b** (120 mg, 0.197 mmol) was separated chirally (separation conditions: Superchiral S-AD (Chiralway), 2cm I.D. × 25 cm Length, 5 µm; mobile phase: carbon dioxide/ethanol/diethylamine = 60/40/0.05 (v/v/v), flow rate: 50 g/min). The fraction with a longer retention time (11.747 minutes) was collected and concentrated under reduced pressure to obtain the title compound **8c** (52 mg).
MS m/z (ESI): 611.0 [M+1].

### Step 3

### 2-[[4-Chloro-2-(trifluoromethyl)phenyl]methyl]-N-[(1R)-1-(4-ethylsulfonylphenyl)-2-(t rideuteromethoxy)ethyl]-1-(2-fluoroethyl)indole-5-carboxamide 8

Compound **8c** (10.5 mg, 0.017 mmol), silver oxide (11 mg, 0.047 mmol), deuterated iodomethane (20 µL, 0.32 mmol) and 1 mL of acetonitrile were mixed and stirred for 48 hours. After addition of another silver oxide (11 mg, 0.047 mmol) and deuterated iodomethane (20 µL, 0.32 mmol), the reaction solution was stirred for 24 hours, and filtrated. The resulting residue was purified by high performance liquid chromatography (Shimadzu SPD-20A high pressure liquid chromatograph, Phenomenex Gemini-NX 5 µM C18 21.2×100 mm chromatographic column, eluent system: trifluoroacetic acid, water and acetonitrile) to obtain the title compound **8** (3 mg).
MS m/z (ESI): 628 [M+1].

### Example 9

### 2-[[4-Chloro-2-(trifluoromethyl)phenyl]methyl]-N-[(1R)-1-(4-ethylsulfonylphenyl)-2-h ydroxyethyl]-1-(1,1,2,2-tetradeutero-2-fluoro-ethyl)indole-5-carboxamide 9

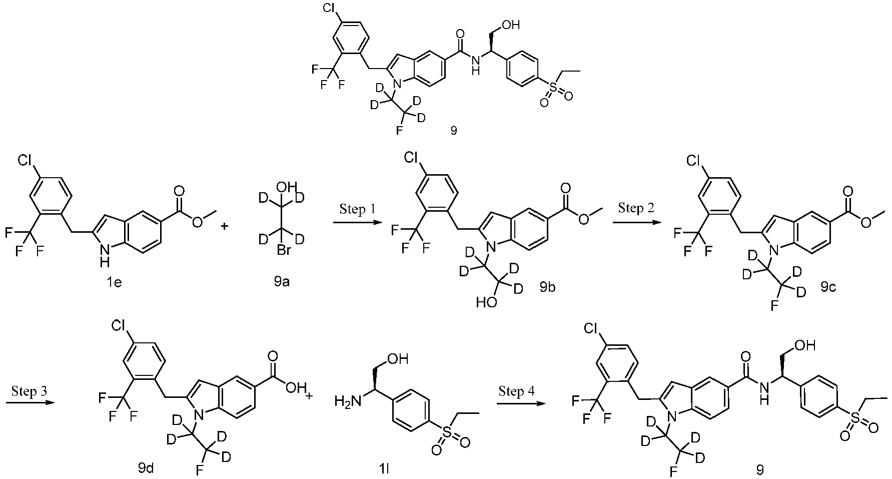

### Step 1

### Methyl 2-[[4-chloro-2-(trifluoromethyl)phenyl]methyl]-1-(1,1,2,2-tetradeutero-2-hydroxy-ethyl )indole-5-carboxylate 9b

Compound **1e** (21.6 mg, 59 µmol) was dissolved in 1 mL of *N*,*N*-dimethylformamide. To the reaction solution was added sodium hydride (23.5 mg, 0.59 mmol). The reaction solution was stirred at room temperature for 45 minutes. After addition of compound 2-bromo-1,1,2,2- tetradeutero-ethanol **9a** (17 mg, 172 µmol), the reaction solution was warmed up to 50°C and stirred for 3 hours. After addition of another compound **9a** (17 mg, 172 µmol), the reaction solution was stirred at 50°C for 16 hours. The reaction solution was cooled and filtrated. The filtrate was concentrated under reduced pressure, and the resulting residue was purified by silica gel column chromatography with eluent system B to obtain the title compound **9b** (1 mg, yield: 4%).
MS m/z (ESI): 415.1 [M+1].

### Steps 2-4

### 2-[[4-Chloro-2-(trifluoromethyl)phenyl]methyl]-N-[(1R)-1-(4-ethylsulfonylphenyl)-2-h ydroxyethyl]-1-(1,1,2,2-tetradeutero-2-fluoro-ethyl)indole-5-carboxamide 9

In accordance with the synthetic route in Steps 7-9 of Example **1**, the starting compound **1i** was replaced with compound **9b**, accordingly, the title compound **9** was prepared.

### Example 10

### 2-[[4-Chloro-2-(trifluoromethyl)phenyl]methyl]-N-[(1R)-1-(4-ethylsulfonylphenyl)-2-h ydroxyethyl]-1-(1,1,2,2,2-pentadeuteroethyl)indole-5-carboxamide 10

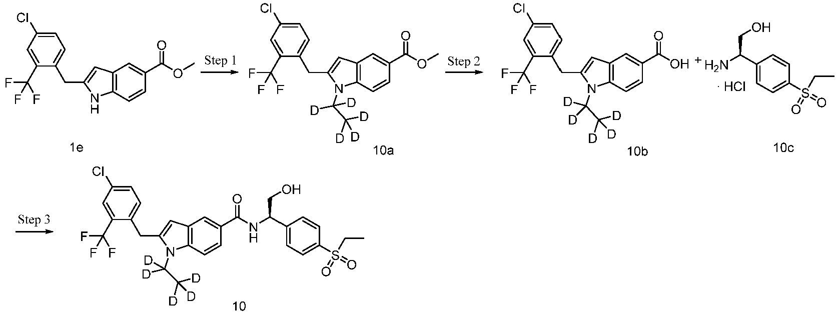

### Step 1

### Methyl 2-[[4-chloro-2-(trifluoromethyl)phenyl]methyl]-1-(1,1,2,2,2-pentadeuteroethyl)indole-5 -carboxylate 10a

In accordance with the synthetic route in Step 5 of Example **3**, the starting compound 1-bromo-2-fluoroethane was replaced with 1,1,1,2,2-pentadeutero-2-iodo-ethane, accordingly, the title compound **10a** (8 mg) was prepared.
MS m/z (ESI): 401 [M+1].

### Step 2

### 2-[[4-Chloro-2-(trifluoromethyl)phenyl]methyl]-N-[(1R)-1-(4-ethylsulfonylphenyl)-2-h ydroxyethyl]-1-(1,1,2,2,2-pentadeuteroethyl)indole-5-carboxamide 10

In accordance with the synthetic route in Steps 8-9 of Example **1**, the starting compound **1j** was replaced with compound **10a**, **1l** was replaced with compound **10c** (prepared according to the method disclosed in the patent application "WO2017024018"), accordingly, the title compound **10** (1.5 mg) was prepared.
MS m/z (ESI): 598 [M+1].

### Example 11

### 2-[[4-Chloro-2-(trifluoromethyl)phenyl]methyl]-1-(1,1-dideuteroethyl)-N-[(1R)-1-(4-eth ylsulfonylphenyl)-2-hydroxyethyl]indole-5-carboxamide 11

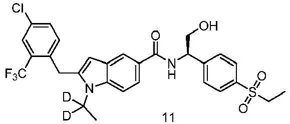

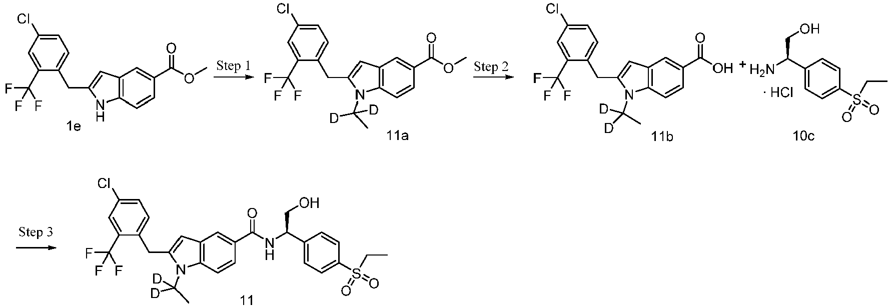

In accordance with the synthetic route in Example **10**, the starting compound 1,1,1,2,2-pentadeutero-2-iodo-ethane was replaced with compound 1,1-dideutero-iodoethane, accordingly, the title compound **11** (1.5 mg) was prepared.
MS m/z (ESI): 595 [M+1].

### Example 12

### 2-[[4-Chloro-2-(trifluoromethyl)phenyl]methyl]-N-[(1R)-1-(4-ethylsulfonylphenyl)-2-h ydroxyethyl]-1-(1,1,2,2-tetradeutero-2-hydroxy-ethyl)indole-5-carboxamide 12

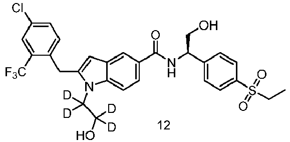

In accordance with the synthetic route in Step 9 of Example **1**, the starting compound **1k** was replaced with compound **9b**, accordingly, the title compound **12** (1.5 mg) was prepared.
MS m/z (ESI): 613 [M+1].

### Example 13

### 2-[[4-Chloro-2-(trifluoromethyl)phenyl]methyl]-1-(2,2-dideutero-2-fluoro-ethyl)-N-[(1 R)-1-(5-ethylsulfonyl-2-pyridinyl)-2-hydroxy-ethyl]indole-5-carboxamide 13

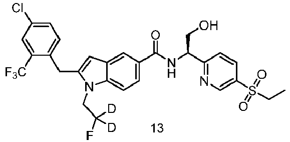

In accordance with the synthetic route in Step 9 of Example **1**, the starting compound **1k** was replaced with compound (*R*)-2-amino-2-(5-(ethylsulfonyl)pyridin-2-yl)ethanol (prepared according to the method disclosed in the patent application "US20160122318"), accordingly, the title compound **13** was prepared.

The following Examples 14-22 can be carried out from corresponding starting compounds in accordance with synthetic routes similar to that in Example 6.

### Example 14

### 1-(2-Fluoroethyl)-2-[[4-fluoro-2-(trifluoromethyl)phenyl]methyl]-N-[(1R)-2-hydroxy-1-[4-(trideuteromethylsulfonyl)phenyl]ethyl]indole-5-carboxamide 14

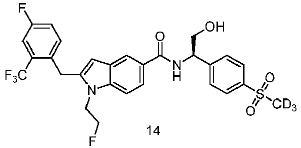

### Example 15

### 2-[[4-Chloro-2-(trifluoromethyl)phenyl]methyl]-1-(2-fluoroethyl)-N-[(1R)-2-hydroxy-1 -[5-(1,1,2,2,2-pentadeuteroethylsulfonyl)-2-pyridinyl]ethyl]indole-5-carboxamide 15

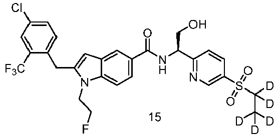

### Example 16

### 1-(2-Fluoroethyl)-2-[[4-fluoro-2-(trifluoromethyl)phenyl]methyl]-N-[(1R)-2-hydroxy-1-[5-(1,1,2,2,2-pentadeuteroethylsulfonyl)-2-pyridinyl]ethyl]indole-5-carboxamide 16

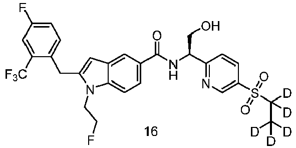

### Example 17

### 1-Cyclopropyl-N-[(1R)-2-hydroxy-1-[4-(1,1,2,2,2-pentadeuteroethylsulfonyl)phenyl]eth yl]-2-[[2-(trifluoromethyl)-3-pyridinyl]methyl]indole-5-carboxamide 17

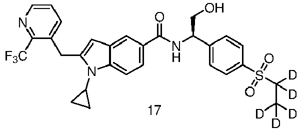

### Example 18

### 2-[[4-Chloro-2-(trifluoromethyl)phenyl]methyl]-1-(2-fluoroethyl)-N-[(1R)-2-hydroxy-1 -[5-(trideuteromethylsulfonyl)-2-pyridinyl]ethyl]indole-5-carboxamide 18

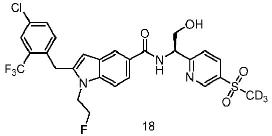

### Example 19

### 2-[4-Chloro-2-(trifluoromethyl)phenyl]methyl]-1-cyclopropyl-N-[(1R)-2-hydroxy-1-[4-(1,1,2,2,2-pentadeuteroethylsulfonyl)phenyl]ethyl]indole-5-carboxamide 19

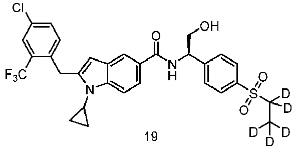

### Example 20

### 2-[[4-Chloro-2-(trifluoromethyl)phenyl]methyl]-1-(2-hydroxyethyl)-N-[(1R)-2-hydroxy -1-[4-(1,1,2,2,2-pentadeuteroethylsulfonyl)phenyl]ethyl]indole-5-carboxamide 20

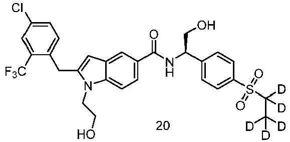

### Example 21

### 2-[[4-Chloro-2-(trifluoromethyl)phenyl]-dideutero-methyl]-N-[(1R)-1-(4-ethylsulfonylp henyl)-2-hydroxy-ethyl]-1-(2-fluoroethyl)indole-5-carboxamide 21

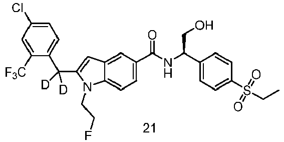

### Example 22

### 2-[[4-Chloro-2-(trifluoromethyl)phenyl]methyl]-N-[(1R)-2,2-dideutero-1-(4-ethylsulfon ylphenyl)-2-hydroxyethyl]-1-(2-fluoroethyl)indole-5-carboxamide 22

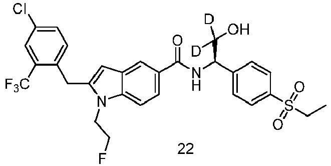

### Examples 23 and 24

### 1-(2-Fluoroethyl)-2-[[4-fluoro-2-(trifluoromethyl)phenyl]methyl]-N-[(1R)-2-hydroxy-1-[4-(1,1,2,2,2-pentadeuteroethylsulfonyl)phenyl]ethyl]indole-5-carboxamide 23

### 1-(2-Fluoroethyl)-2-[[4-fluoro-2-(trifluoromethyl)phenyl]methyl]-N-[(1S)-2-hydroxy-1-[4-(1,1,2,2,2-pentadeuteroethylsulfonyl)phenyl]ethyl]indole-5-carboxamide 24

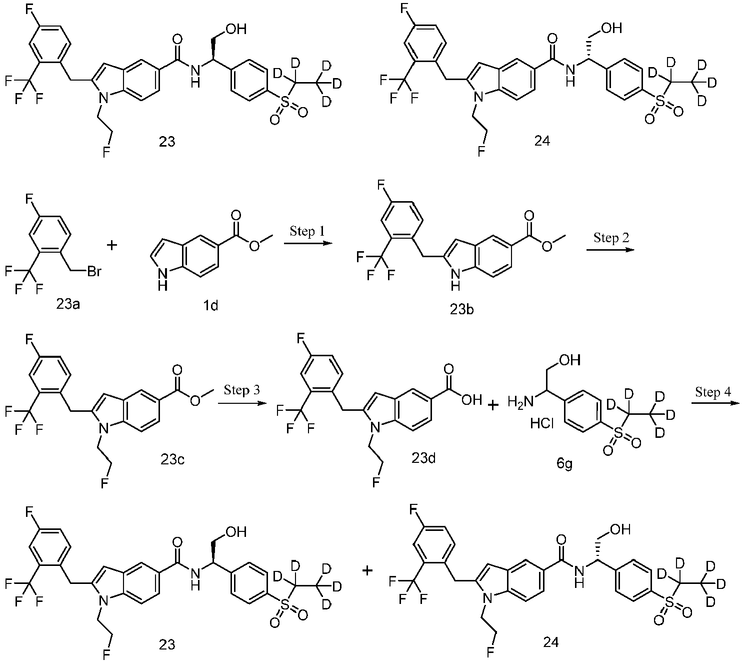

### Step 1

Methyl 2-(4-fluoro-2-(trifluoromethyl)benzyl)-1*H*-indole-5-carboxylate **23b** Compound **1d** (1.3 g, 7.42 mmol) and 1-(bromomethyl)-4-fluoro-2-(trifluoromethyl)benzene **23a** (2.29 g, 8.90 mmol) were dissolved in 20 mL of *N*,*N*-dimethylacetamide. To the reaction solution were added bis(acetonitrile)palladium (II) dichloride (385.03 mg, 1.48 mmol), bicyclo[2.2.1]-2-heptene (698.67 mg, 7.42 mmol) and sodium carbonate (1.57 g, 14.84 mmol). The reaction solution was heated to 80°C and stirred for 17 hours under an argon atmosphere. The reaction solution was cooled and filtrated. The filtrate was concentrated under reduced pressure, and the resulting residue was purified by silica gel column chromatography with eluent system B to obtain the title compound **23b** (2.0 g, yield: 76.72%).
MS m/z (ESI): 352.1 [M+1].

### Step 2

### Methyl 2-(4-fluoro-2-(trifluoromethyl)benzyl)-1-(2-fluoroethyl)-1H-indole-5-carboxylate 23c

Compound **23b** (0.14 g, 398.53 µmol) and 1-bromo-2-fluoroethane (151.8 mg, 1.20 mmol) were dissolved in 10 mL of *N*,*N*-dimethylformamide. To the reaction solution was added cesium carbonate (389.54 mg, 1.20 mmol). After stirring for 1 hour at 100°C under microwave, the reaction solution was cooled and filtrated. The filtrate was concentrated under reduced pressure, and the resulting residue was purified by silica gel column chromatography with eluent system B to obtain the title compound **23c** (0.135 g, yield: 85.3%).
MS m/z (ESI): 398.0 [M+1].

### Step 3

### 2-(4-Fluoro-2-(trifluoromethyl)benzyl)-1-(2-fluoroethyl)-1H-indole-5-carboxylic acid 23d

Compound **23c** (0.13 g, 339.76 µmol) was dissolved in 15 mL of methanol. To the reaction solution was added 1.5 mL of 4*N* sodium hydroxide solution. The reaction solution was stirred under reflux for 1 hour, and then cooled to room temperature. 1*M* hydrochloric acid was added dropwise to adjust the pH to 3∼4. After addition of 20 mL of water and 20 mL of ethyl acetate, the reaction solution was extracted with ethyl acetate (20mL×2). The organic phases were combined, dried over anhydrous sodium sulfate, and filtrated. The filtrate was concentrated under reduced pressure, and the resulting residue was purified by silica gel column chromatography with eluent system A to obtain the title compound **23d** (0.125 g, yield: 96.0%).
MS m/z (ESI): 384.0 [M+1].

### Step 4

### 1-(2-Fluoroethyl)-2-[[4-fluoro-2-(trifluoromethyl)phenyl]methyl]-N-[(1R)-2-hydroxy-1-[4-(1,1,2,2,2-pentadeuteroethylsulfonyl)phenyl]ethyl]indole-5-carboxamide 23

### 1-(2-Fluoroethyl)-2-[[4-fluoro-2-(trifluoromethyl)phenyl]methyl]-N-[(1S)-2-hydroxy-1-[4-(1,1,2,2,2-pentadeuteroethylsulfonyl)phenyl]ethyl]indole-5-carboxamide 24

Compound **23d** (250 mg, 652.21 µmol) was dissolved in 10 mL of *N*,*N*-dimethylformamide. To the reaction solution were added compound **6g** (211.93 mg, 782.65 µmol) and *N*,*N*-diisopropylethylamine (168.58 mg, 1.30 mmol), followed by 2-(7-azabenzotriazole)-*N*,*N*,*N*',*N*'-tetramethyluronium hexafluorophosphate (371.76 mg, 978.32 µmol). The reaction solution was stirred at room temperature for 2 hours, and then concentrated under reduced pressure. The resulting residue was purified by high performance liquid chromatography (Waters 2767-SQ high pressure liquid chromatograph, sunfire OBD, 150^{∗}19 mm 5nfi chromatographic column, eluent system: ammonium bicarbonate, water and acetonitrile) to obtain the crude compound (140 mg). The crude product was separated chirally (separation conditions: chiral preparative column Amylose-1 20^{∗}250mm, 5 µm; mobile phase: ethanol/*n*-hexane = 40/60 (v/v), flow rate: 20 mL/minute). The corresponding fractions were collected and concentrated under reduced pressure to obtain the title compounds **23** (100 mg, yield: 25.57%) and **24** (10 mg, yield: 2%).

Compound **23**:
MS m/z (ESI): 600.0 [M+1].

Chiral HPLC analysis: retention time 8.384 minutes, chiral purity: 100% (chromatographic column: Phenomenex Lux Cellulose-1 (OD) 150^{∗}4.6mm, 5 µm (equipped with a guard column); mobile phase: *n*-hexane/ethanol (containing 0.1% of diethylamine) = 60/40 (v/v)).
¹HNMR(400MHz, CDCl₃) δ 8.12 (s, 1H), 7.94-7.92 (d, 2H), 7.77-7.74 (d, 1H), 7.65-7.63 (d, 2H), 7.50-7.47 (d, 1H),7.38-7.36 (d, 1H), 7.29-7.27 (m, 1H), 7.13-7.11 (m, 2H), 6.32 (s, 1H), 5.41-5.37 (m, 1H), 4.70-4.69 (m, 1H), 4.58-4.56 (m, 1H), 4.39-4.37(m, 1H), 4.35 (s, 2H), 4.32-4.30 (m, 1H), 4.08-4.05 (dd, 1H), 4.01-3.97 (dd, 1H).

Compound **24**:
MS m/z (ESI): 600.0 [M+1].

Chiral HPLC analysis: retention time 9.978 minutes, chiral purity: 100% (chromatographic column: Phenomenex Lux Cellulose-1 (OD) 150^{∗}4.6mm, 5 µm (equipped with a guard column); mobile phase: *n*-hexane/ethanol (containing 0.1% of diethylamine) = 60/40 (v/v)).
¹HNMR(400MHz, CDCl₃) δ 8.13 (s, 1H), 7.93-7.91 (d, 2H), 7.77-7.74 (d, 1H), 7.65-7.63 (d, 2H), 7.50-7.47 (d, 1H),7.38-7.36 (d, 1H), 7.29-7.27 (m, 1H), 7.13-7.11 (m, 2H), 6.32 (s, 1H), 5.41-5.37 (m, 1H), 4.70-4.69 (m, 1H), 4.58-4.56 (m, 1H), 4.39-4.37(m, 1H), 4.35 (s, 2H), 4.32-4.30 (m, 1H), 4.10-4.07 (dd, 1H), 4.01-3.97 (dd, 1H).

### Biological Assay

The present invention will be further described with reference to the following test examples, but the examples should not be considered as limiting the scope of the present invention.

Test Example 1. Determination of the effect of the compounds of the present invention on the *in vitro* activity of RORγ

### I. Experimental materials and instruments

1. LanthaScreen® TR-FRET RORγ co-activation system (Life Technologies)
2. RORγ LBD (AB Vector)
3. DMSO (SigmaAldrich)
4. Microplate reader (Tecan)

### II. Experimental procedures

The effect of the compounds of the present invention on RORγ activity was screened with a LanthaScreen TR-FRET (Time Resolved Fluorescence Resonance Energy Transfer) RORγ co-activation system.

A complete buffer D (complete TR-FRET Coregulator) (Life Technologies) was formulated first, containing a final concentration of 5 mM DTT. The final concentration of DMSO was 2%. The test compound was serially diluted to 2x final concentration in the complete buffer D containing 2% of DMSO, and the maximum dose was 60 µm. The test compound was added to the test wells of a 384-well plate (PerkinElmer) in 10 µl/well. Two parallel control wells were set up for each test compound at the same concentration. 4X RORγ LBD (AB Vector) was formulated. RORγ LBD was diluted with the complete buffer D to a concentration of 1 ng/µL, and added to the test wells of the 384-well plate in 5 µl/well. The negative control well was 5 µL of complete buffer D without RORγ LBD. A mixed solution comprising 0.6 µM of fluorescein-D22 (4X) and 8 nM of terbium (Tb)-labeled anti-GST antibody (4X) (Life Technologies) was formulated with the complete buffer D, and 5 µL of the mixed solution was added to the 384-well plate. The total reaction system was 20 µL. The 384-well plate was gently shaken on a shaker, and incubated at room temperature in the dark for 2-4 hours.

Fluorescence readings were determined with Tecan Infinite M1000. The logarithmic curve of the ratio of the emission wavelength of 520 nm/495 nm to the concentration of the compound was plotted by GraphPad Prism 6.0 software. EC₅₀ value of the test compound was calculated.

The effect of the compounds of the present invention on the *in vitro* activity of RORγ was determined by the above test, and the resulting EC₅₀ values are shown in Table 1.

**Table 1 EC₅₀ values of the compounds of the present invention on the in vitro activity of RORγ**

| Example No. | EC₅₀ (nM) | Emax(%) |
|---|---|---|
| 1 | 9 | 77% |
| 2 | 16 | 89% |
| 3 | 17 | 98% |
| 4 | 5 | 98% |
| 5 | 9 | 73% |
| 6 | 33 | 102% |
| 8 | 34 | 85% |
| 11 | 21 | 84% |
| 13 | 9 | 77% |

Conclusion: The compounds of the present invention have a significant agonistic effect on the *in vitro* activity of RORγ.

Test Example 2. Determination of the activity of the compounds of the present invention on IL-17A by enzyme-linked immune quantitative assay

### I. Experimental materials and instruments

1. Human peripheral blood mononuclear cells (PBMC) (Zenbio)
2. Lymphocyte culture medium (Zenbio)
3. TexMACS (Miltenyi Biotec)
4. Human cytostim (Miltenyi Biotec)
5. Human IL-17 enzyme-linked immunosorbent kit (R&D System)
6. CO₂ incubator (Fisher Scientific)
7. Centrifuge (Fisher Scientific)
8. Microplate reader (Tecan)

### II. Experimental procedures

Frozen human peripheral blood mononuclear cells (PBMC) were rapidly resuscitated in pre-warmed lymphocyte culture medium, and centrifuged at 1000 rpm for 10 min. The cell culture supernatant was removed. The cells were gently suspended in TexMACS medium and counted. The T cell activation reagent cytostim (10 µl/ml) was added in proportion to the cell suspension. Then, the cells were seeded in a 96-well cell culture plate at a density of 1×10⁵ peripheral blood mononuclear cells/well. The test compounds were diluted in gradient with TexMACS medium, and added respectively to the test wells, with 2-3 parallel wells per group. A negative control well containing only cells without cytostim was provided to obtain the background reading. The cell culture plate was placed in a incubator at 5% carbon dioxide, 37°C to incubate for 3 days. The cell culture supernatant was collected 3 days after drug treatment, and centrifuged to remove the suspension. Then, IL-17A in the supernatant was quantified with IL-17A enzyme-linked immunosorbent kit. EC₅₀ values of the test compounds were calculated with GraphPad Prism 6.0.

The effect of the compounds of the present invention on IL-17A by enzyme-linked immune quantitative assay was determined by the above test, and the resulting EC₅₀ values are shown in Table 2.

**Table 2 EC₅₀ values of the compounds of the present invention on IL-17A by enzyme-linked immune quantitative assay**

| Example No. | EC₅₀ (nM) | Emax(%) |
|---|---|---|
| 1 | 22 | 88% |
| 3 | 103 | 102% |
| 4 | 20 | 79% |
| 5 | 32 | 82% |
| 6 | 37 | 100% |
| 13 | 22 | 88% |

Conclusion: The compounds of the present invention have a significant regulation effect on IL-17A by enzyme-linked immune quantitative assay.

### Pharmacokinetics Evaluation

Test Example 3. Pharmacokinetics assay of the compound of the present invention in mice

### 1. Abstract

Mice were used as test animals. The drug concentration in plasma at different time points was determined by LC/MS/MS method after intragastrical administration of the compounds of Example 1 and Example 6 to mice. The pharmacokinetic behavior of the compounds of the present invention was studied and evaluated in mice.

### 2. Test protocol

### 2.1 Test compounds

Compounds of Example 1 and Example 6.

### 2.2 Test animals

A group of eighteen C57 mice (female, equally divided into two groups) were purchased from Shanghai Jiesijie Laboratory Animal Co., LTD, with Certificate No.: SCXK (Shanghai) 2013-0006.

### 2.3 Preparation of the test compound

A certain amount of the test compound was weighed, and added with 5% by volume of DMSO, 5% by volume of tween 80 and 90% by volume of normal saline to prepare a 0.1 mg/mL colorless, clear and transparent solution.

### 2.4 Administration

After an overnight fast, C57 mice were intragastrically administered the test compound at an administration dose of 2.0 mg/kg and an administration volume of 0.2 mL/10 g.

### 3. Process

The mice were intragastrically administered the compounds of Example 1 and Example 6. 0.1 ml of blood was taken (from 3 animals at each time point) before administration and at 0.25, 0.5, 1.0, 2.0, 4.0, 6.0, 8.0, 11.0 and 24.0 hours after administration. The samples were stored in heparinized tubes, and centrifuged for 10 minutes at 3500 rpm to separate the blood plasma. The plasma samples were stored at -20°C.

The content of the test compound in the plasma of mice after intragastrical administration of the test compound at different concentrations was determined: 25 µL of mouse plasma at each time after administration was taken, added with 80 µL of the internal standard solution of camptothecin (100 ng/mL) and 200 µL of acetonitrile, vortex-mixed for 5 minutes, and centrifuged for 10 minutes (3600 rpm). 1 µL of the supernatant was taken from the plasma samples for LC/MS/MS analysis.

### 4. Results of pharmacokinetic parameters

Pharmacokinetic parameters of the compounds of the present invention are shown below:

| No. | Pharmacokinetics assay in mice (2 mg/kg) | | | | | |
|---|---|---|---|---|---|---|
| | Plasma concentration | Area under curve | Half-life | Residence time | Clearance | Apparent distribution volume |
| | Cmax (ng /mL) | AUC (ng /mL^{∗}h) | T1/2 (h) | MRT (h) | CLz/F (ml/min/kg) | Vz/F (ml/kg) |
| Example 1 | 3713 | 40447 | 9.08 | 12 | 0.82 | 648 |
| Example 6 | 2730 | 31556 | 6.29 | 9.13 | 1.06 | 575 |

Conclusion: The compound of the present invention is well absorbed, and has a pharmacokinetic advantage.

### Pharmacodynamic Evaluation

Test Example 4. Efficacy of RORγ agonist in isotype MC38 colorectal tumor mice model

### 1. Experimental purpose

The inhibition effect of the compound of Example 1 on MC38 colorectal tumor growth was evaluated in MC38 mice model.

### 2. Experimental method and experimental materials

### 2.1. Test animals and feeding conditions

Experimental female C57BL/6 mice were purchased from Charles River Lab (U.S.A.). The mice weighed 20-25 gram, and were 7-9 weeks old when purchased. The mice (10 mice per cage) were maintained in a constant temperature of 23±1°C, and a humidity of 50-60%, and free access to food and water. The mice were treated and used in accordance with the Institutional Animal Care and Use Committee (IACUC approved guidelines). After the animals were purchased, the test was started after 7 days of adaptive feeding.

### 2.2. Experimental drugs

Compound of Example 1;

Anti-mouse PD-1 (CD279) antibody, purchased from BioXcell (clone RMP1-14; catalog number BP0146);

IgG2a isotype control antibody, purchased from BioXcell (clone 2A3; catalog number BE0089).

### 2.3. Experimental design and experimental method

### 2.3.1. Animal grouping:

After adaptive feeding, the mice were grouped as follows:

| No. | Groups | Mice/group | Administration mode | Administration regimen |
|---|---|---|---|---|
| Group I | IgG2a isotype control antibody plus vehicle control group | 8 | Intraperitoneal injection/oral administration | Q3dx4¹/QDx20² |
| Group II | Anti-mouse PD-1 antibody | 8 | Intraperitoneal injection | Q3dx4 |
| Group III | Compound of Example 1 | 8 | Oral administration | QDx20 |
| Group IV | Anti-mouse PD-1 antibody plus compound of Example 1 | 8 | Intraperitoneal injection/oral administration | Q3dx4/QDx20 |

| | | | | |
|---|---|---|---|---|
| Note: 1. Q3dx4 refers to administration every three days for a total of four times; 2. QDx20 refers to administration once a day for 20 consecutive days; 3. IgG2a and PD-1 antibody were administered by intraperitoneal injection, the compound of Example 1 and vehicle were administered orally. | | | | |

### 2.3.2. Experimental method

Female C57BL/6 mice (20-25 gram, 7-9 weeks old) were used in the experiment. *In vivo* antitumor activity of the compound of Example 1 administered alone or the compound of Example 1 administered in combination with anti-mouse PD-1 antibody was evaluated by detecting the growth of isotype MC38 colorectal tumor in inbred C57BL/6 mice. 500,000 (5×10⁵) MC38 cells were implanted subcutaneously in the right abdomen of each mouse. When the tumor grew to 40-80 mm³ (on Day 6), the mice were grouped randomly into the above four groups. In Group III and IV, the mice were administered the compound of Example 1 (0.5 mg/kg) once a day for 20 consecutive days. During the treatment experiment in Group II and IV, the anti-mouse PD-1 (CD279) antibody (BioXcell) (5 mg/kg) was intraperitoneally injected (i.p.) to the mice bearing MC38 tumor on Day 6, 9, 12 and 15, respectively. Group I (control group) was administered with the vehicle CMC-Na drug formulation and the IgG2a isotype control antibody, wherein the administration mode of the vehicle CMC-Na drug formulation was the same as that of the compound of Example 1, and the administration mode of the IgG2a isotype control antibody was the same as that of the anti-mouse PD-1 (CD279) antibody.

### 2.4. Data presentation:

The tumor volume was measured with a caliper in three dimensions, and then calculated according to the following formula: tumor volume (mm³) = 1 × w × h × 0.5236, wherein 1 represents the length of the tumor, w represents the width of the tumor, and h represents the height of the tumor, in millimeters. Tumor growth inhibition rate TGI% = 100 × (TV_{control} - TVₜᵤₘₒᵣ) / (TV_{control} - TVᵢₙᵢₜᵢₐₗ), wherein TV_{control} = the tumor volume of the control group; TVₜᵤₘₒᵣ = the tumor volume of the treatment group; and TVᵢₙᵢₜᵢₐₗ = the initial tumor volume on Day 6.

### 3. Results and discussion:

As shown in Figure 1, when 0.5 mg/kg of the compound of Example 1 was administered alone, the TGI was 22.5%. When the anti-mouse PD-1 (CD279) antibody (5 mg/kg) was injected alone, the TGI was 39.8%. When administered in combination with the anti-mouse PD-1 monoclonal antibody (5 mg/kg), the compound of Example 1 (administered at 0.5 mg/kg) exhibited a synergistic effect (the TGI was 65.5%). These data indicate that in the isogenic MC38 colorectal tumor model, the administration of the compound of Example 1 alone exhibits an antitumor activity, and the combined administration of the compound of Example 1 and PD-1 antibody exhibits a synergistic effect. These data also indicate that the compound of Example 1 has a biological activity consistent with RORγ activation (rather than inhibition), establishing a novel way of improving the efficacy of immunotherapy.

## Claims

1. A compound of formula (I): 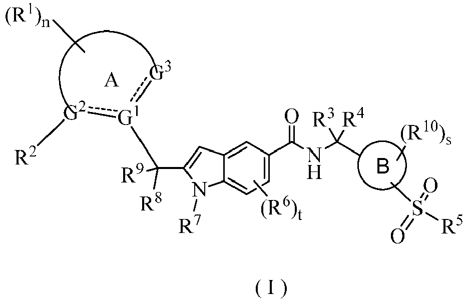 or a tautomer, mesomer, racemate, enantiomer, diastereomer thereof, or mixture thereof, or a pharmaceutically acceptable salt thereof,
wherein:
----- is a double bond or single bond;
G¹, G² and G³ are identical or different and are each independently selected from the group consisting of C, CH, CH₂ and N;
ring A is selected from the group consisting of aryl, heteroaryl, cycloalkyl and heterocyclyl;
ring B is an aryl or heteroaryl;
each R¹ is identical or different and each is independently selected from the group consisting of H atom, D atom, halogen, alkyl, haloalkyl, alkoxy, haloalkoxy, cyano, amino, nitro, hydroxy and hydroxyalkyl;
R² is a haloalkyl;
R³ and R⁴ are identical or different and are each independently selected from the group consisting of H atom, D atom, halogen, alkyl, haloalkyl, alkoxy, haloalkoxy, hydroxyalkyl, cyano, amino, nitro, hydroxy, cycloalkyl, heterocyclyl, aryl and heteroaryl, wherein the alkyl, hydroxyalkyl, cycloalkyl, heterocyclyl, aryl and heteroaryl are each independently optionally substituted by one or more substituents selected from the group consisting of D atom, hydroxy, halogen, alkyl, amino and -OR¹¹;
R⁵ is selected from the group consisting of H atom, alkyl, haloalkyl, amino, hydroxy, hydroxyalkyl, cycloalkyl, heterocyclyl, aryl and heteroaryl, wherein the alkyl, cycloalkyl, heterocyclyl, aryl and heteroaryl are optionally substituted by one or more substituents selected from the group consisting of D atom, halogen, hydroxy, cycloalkyl and heterocyclyl;
each R⁶ is identical or different and each is independently selected from the group consisting of H atom, D atom, halogen, alkyl, haloalkyl, alkoxy, haloalkoxy, cyano, amino, nitro, hydroxy, hydroxyalkyl, cycloalkyl, heterocyclyl, aryl and heteroaryl;
R⁷ is selected from the group consisting of H atom, alkyl, haloalkyl, cycloalkyl, heterocyclyl, aryl and heteroaryl, wherein the alkyl, cycloalkyl, heterocyclyl, aryl and heteroaryl are optionally substituted by one or more substituents selected from the group consisting of D atom, halogen, hydroxy, amino, cyano, nitro, alkoxy, haloalkoxy, -OR¹¹, -C(O)R¹¹, -C(O)OR¹¹, -NR¹²R¹³, -C(O)NR¹²R¹³, -S(O)ₘR¹¹, cycloalkyl and heterocyclyl;
R⁸ and R⁹ are identical or different and are each independently selected from the group consisting of H atom, D atom, halogen, alkyl, haloalkyl, alkoxy, cyano, amino, nitro, hydroxy and hydroxyalkyl;
each R¹⁰ is identical or different and each is independently selected from the group consisting of H atom, D atom, halogen, alkyl, haloalkyl, alkoxy, haloalkoxy, cyano, amino, nitro, hydroxy, hydroxyalkyl, cycloalkyl, heterocyclyl, aryl and heteroaryl;
R¹¹ is selected from the group consisting of H atom, D atom, alkyl, haloalkyl, hydroxyalkyl, cycloalkyl and heterocyclyl, wherein the alkyl, hydroxyalkyl, cycloalkyl and heterocyclyl are optionally substituted by one or more substituents selected from the group consisting of D atom, halogen, hydroxy, cycloalkyl and heterocyclyl;
R¹² and R¹³ are identical or different and are each independently selected from the group consisting of H atom, D atom, alkyl, haloalkyl, hydroxy and hydroxyalkyl, wherein the alkyl and hydroxyalkyl are optionally substituted by one or more substituents selected from the group consisting of D atom, halogen, hydroxy, cycloalkyl and heterocyclyl;
provided that the compound of formula (I) comprises at least one D atom;
m is 0, 1 or 2;
n is 0, 1, 2, 3 or 4;
s is 0, 1, 2 or 3; and
t is 0, 1, 2 or 3.

2. The compound of formula (I) according to claim 1, wherein ring A is selected from the group consisting of phenyl, pyridyl, imidazolyl, pyrazolyl, piperidinyl and morpholinyl; and ring B is a phenyl or pyridyl.

3. The compound of formula (I) according to claim 1 or 2, being a compound of formula (II): 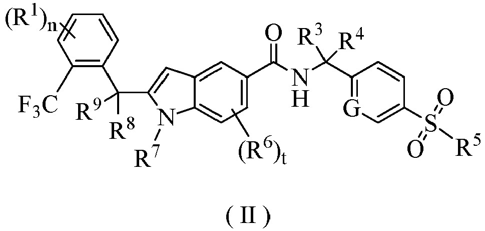 wherein:
G is CH or N; and
R¹, R³∼R⁹, n and t are as defined in claim 1.

4. The compound of formula (I) according to any one of claims 1 to 3, wherein R⁴ is a H atom or D atom; R³ is selected from the group consisting of H atom, D atom and alkyl, wherein the alkyl is optionally substituted by one or more substituents selected from the group consisting of D atom, hydroxy, halogen, amino and -OR¹¹; and R¹¹ is as defined in claim 1.

5. The compound of formula (I) according to any one of claims 1 to 4, wherein R⁷ is selected from the group consisting of alkyl, haloalkyl, cycloalkyl and heterocyclyl, wherein the alkyl is optionally substituted by one or more substituents selected from the group consisting of D atom, halogen, hydroxy, amino, cyano, nitro, alkoxy, haloalkoxy and hydroxyalkyl.

6. The compound of formula (I) according to any one of claims 1 to 5, wherein R⁸ and R⁹ are identical or different and are each independently selected from the group consisting of H atom and D atom.

7. The compound of formula (I) according to any one of claims 1 to 6, being a compound of formula (III): 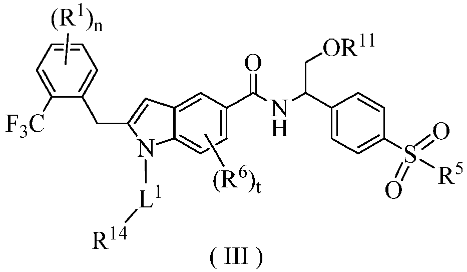 wherein:
L¹ is an alkylene, wherein the alkylene is optionally substituted by one or more substituents selected from the group consisting of halogen and D atom;
R¹⁴ is selected from the group consisting of D atom, halogen, hydroxy, amino, cyano, nitro, alkoxy, haloalkoxy and hydroxyalkyl; and
R¹, R⁵, R⁶, R¹¹, n and t are as defined in claim 1.

8. The compound of formula (I) according to any one of claims 1 to 7, being a compound of formula (IV): 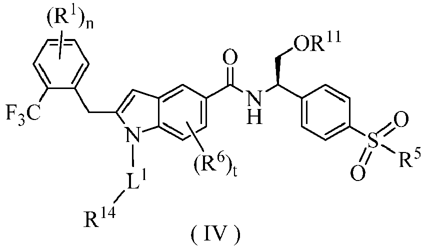 wherein:
L¹ is an alkylene, wherein the alkylene is optionally substituted by one or more substituents selected from the group consisting of halogen and D atom;
R¹⁴ is selected from the group consisting of D atom, halogen, hydroxy, amino, cyano, nitro, alkoxy, haloalkoxy and hydroxyalkyl; and
R¹, R⁵, R⁶, R¹¹, n and t are as defined in claim 1.

9. The compound of formula (I) according to any one of claims 1 to 8, wherein each R¹ is identical or different and each is independently selected from the group consisting of H atom, D atom, halogen and alkyl.

10. The compound of formula (I) according to any one of claims 1 to 9, wherein R⁵ is an alkyl, wherein the alkyl is optionally substituted by one or more substituents selected from the group consisting of D atom, halogen and hydroxy.

11. The compound of formula (I) according to any one of claims 1 to 10, wherein each R⁶ is identical or different and each is independently selected from the group consisting of H atom, D atom and halogen.

12. The compound of formula (I) according to any one of claims 1 to 11, selected from the group consisting of: 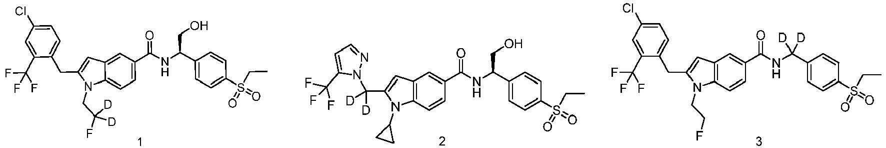 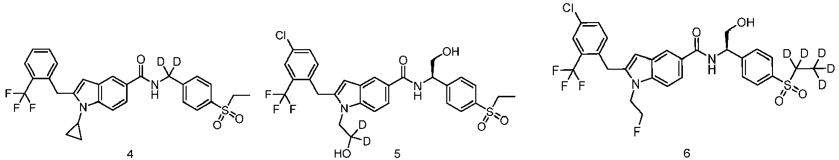 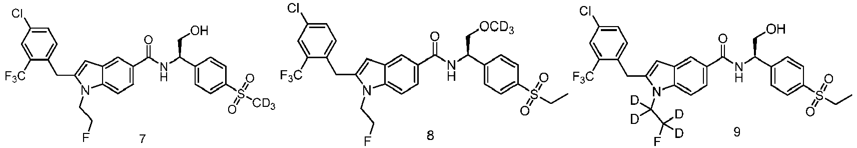 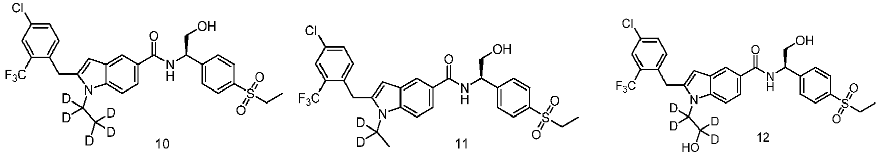 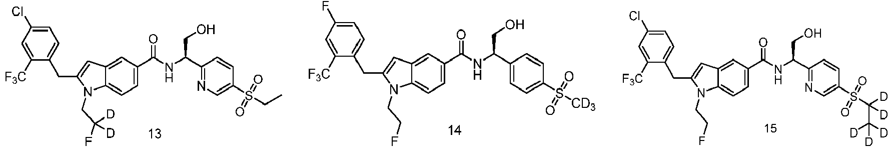 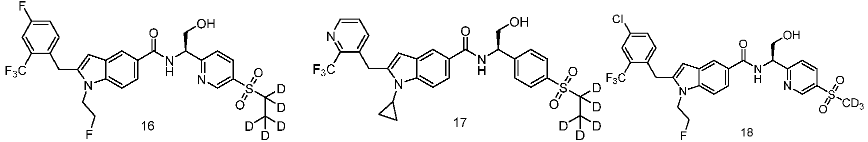 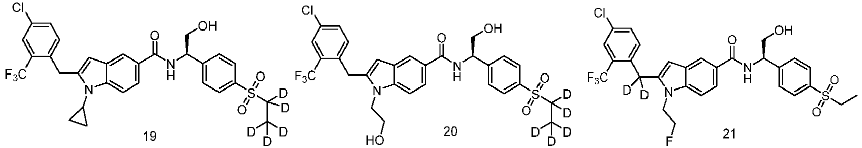 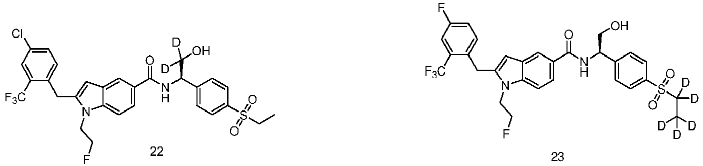 and

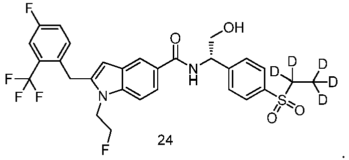

13. A compound of formula (V): 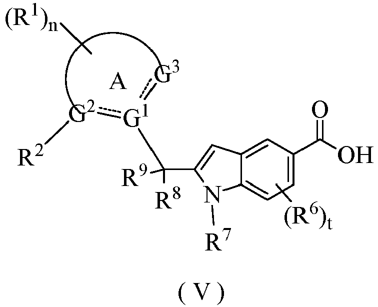 or a tautomer, mesomer, racemate, enantiomer, diastereomer thereof, or mixture thereof, or a pharmaceutically acceptable salt thereof,
wherein:
----- is a double bond or single bond;
G¹, G² and G³ are identical or different and are each independently selected from the group consisting of C, CH, CH₂ and N;
ring A is selected from the group consisting of aryl, heteroaryl, cycloalkyl and heterocyclyl;
each R¹ is identical or different and each is independently selected from the group consisting of H atom, D atom, halogen, alkyl, haloalkyl, alkoxy, haloalkoxy, cyano, amino, nitro, hydroxy and hydroxyalkyl;
R² is a haloalkyl;
each R⁶ is identical or different and each is independently selected from the group consisting of H atom, D atom, halogen, alkyl, haloalkyl, alkoxy, haloalkoxy, cyano, amino, nitro, hydroxy, hydroxyalkyl, cycloalkyl, heterocyclyl, aryl and heteroaryl;
R⁷ is selected from the group consisting of H atom, alkyl, haloalkyl, cycloalkyl, heterocyclyl, aryl and heteroaryl, wherein the alkyl, cycloalkyl, heterocyclyl, aryl and heteroaryl are optionally substituted by one or more substituents selected from the group consisting of D atom, halogen, hydroxy, amino, cyano, nitro, alkoxy, haloalkoxy, -OR¹¹, -C(O)R¹¹, -C(O)OR¹¹, -NR¹²R¹³, -C(O)NR¹²R¹³, -S(O)ₘR¹¹, cycloalkyl and heterocyclyl;
R⁸ and R⁹ are identical or different and are each independently selected from the group consisting of H atom, D atom, halogen, alkyl, haloalkyl, alkoxy, cyano, amino, nitro, hydroxy and hydroxyalkyl;
R¹¹ is selected from the group consisting of H atom, D atom, alkyl, haloalkyl, hydroxyalkyl, cycloalkyl and heterocyclyl, wherein the alkyl, hydroxyalkyl, cycloalkyl and heterocyclyl are optionally substituted by one or more substituents selected from the group consisting of D atom, halogen, hydroxy, cycloalkyl and heterocyclyl;
R¹² and R¹³ are identical or different and are each independently selected from the group consisting of H atom, D atom, alkyl, haloalkyl, hydroxy and hydroxyalkyl, wherein the alkyl and hydroxyalkyl are optionally substituted by one or more substituents selected from the group consisting of D atom, halogen, hydroxy, cycloalkyl and heterocyclyl;
m is 0, 1 or 2;
n is 0, 1, 2, 3 or 4; and
t is 0, 1, 2 or 3.

14. The compound of formula (V) according to claim 13, wherein at least one of substituents R⁷, R⁸ and R⁹ comprises one or more D atoms.

15. The compound of formula (V) according to claim 13, selected from the group consisting of:

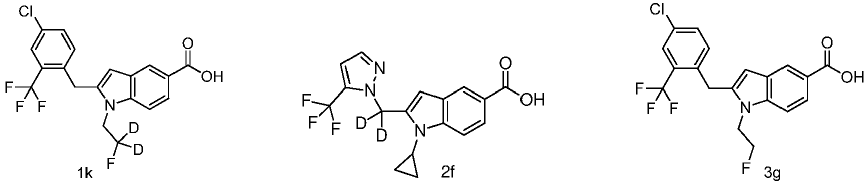

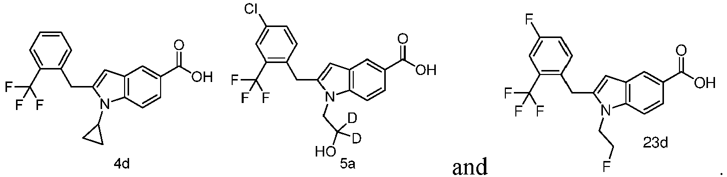

16. A method for preparing the compound of formula (I) according to claim 1, comprising a step of: 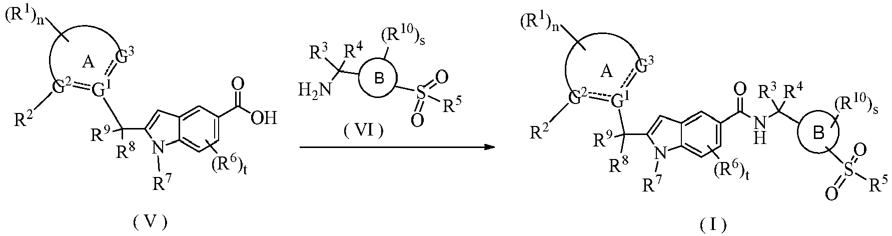 subjecting a compound of formula (V) and a compound of formula (VI) or a pharmaceutically acceptable salt thereof to a condensation reaction to obtain the compound of formula (I),
wherein:
-----, ring A, ring B, G¹∼G³, R¹∼R¹⁰, n, s and t are as defined in claim 1.

17. A pharmaceutical composition, comprising a therapeutically effective amount of the compound of formula (I) according to any one of claims 1 to 12, and one or more pharmaceutically acceptable carriers, diluents or excipients.

18. The pharmaceutical composition according to claim 17, further comprising an anti-PD-1 antibody.

19. Use of the compound of formula (I) according to any one of claims 1 to 12 or the pharmaceutical composition according to claim 17 in the preparation of a ROR agonist.

20. Use of the compound of formula (I) according to any one of claims 1 to 12 or the pharmaceutical composition according to claim 17 in the preparation of a medicament for preventing and/or treating tumor or cancer.

21. Use of the compound of formula (I) according to any one of claims 1 to 12 or the pharmaceutical composition according to claim 17 in combination with an anti-PD-1 antibody in the preparation of a medicament for preventing and/or treating tumor or cancer.

22. The use according to claim 20 or 21, wherein the tumor or cancer is a solid tumor or blood tumor, preferably selected from the group consisting of non-Hodgkin's lymphoma, diffuse large B-cell lymphoma, follicular lymphoma, synovial sarcoma, breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, glioma, glioblastoma, hepatocellular carcinoma, papillary renal carcinoma, head and neck tumor, leukemia, lymphoma, myeloma and non-small cell lung cancer.
